(19)

Europäisches Patentamt

European Patent Office

Office européen des brevets

(11)  **EP 4 205 748 A1**

(12)  **EUROPEAN PATENT APPLICATION**
published in accordance with Art. 153(4) EPC

(43) Date of publication:
**05.07.2023 Bulletin 2023/27**

(21) Application number: **21860266.2**

(22) Date of filing: **20.08.2021**

(51) International Patent Classification (IPC):
*A61K 31/704* (2006.01)    *A61K 36/904* (2006.01)
*A61K 36/899* (2006.01)    *A61K 36/8988* (2006.01)
*A61K 36/898* (2006.01)    *A61K 36/8968* (2006.01)
*A61K 36/8966* (2006.01)    *A61K 36/8965* (2006.01)
*A61K 36/8945* (2006.01)    *A61K 36/725* (2006.01)
*A61K 36/57* (2006.01)    *A61K 36/258* (2006.01)
*A61P 37/04* (2006.01)    *A61P 35/00* (2006.01)
*A61P 39/00* (2006.01)    *A61K 35/64* (2015.01)
*A61K 36/734* (2006.01)

(86) International application number:
**PCT/CN2021/113658**

(87) International publication number:
**WO 2022/042428 (03.03.2022 Gazette 2022/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **25.08.2020   CN 202010861400**

(71) Applicant: **Dalian Fusheng Natural Medicine
Development Co., Ltd.
Dalian, Liaoning 116100 (CN)**

(72) Inventors:
• **WANG, Shuo
  Dalian, Liaoning 116600 (CN)**

• **FU, Li
  Dalian, Liaoning 116600 (CN)**
• **LIU, Yang
  Dalian, Liaoning 116600 (CN)**
• **LU, Mingming
  Dalian, Liaoning 116600 (CN)**
• **LIN, Rongxin
  Dalian, Liaoning 116600 (CN)**
• **FU, Wenfei
  Dalian, Liaoning 116600 (CN)**
• **FENG, Xue
  Dalian, Liaoning 116600 (CN)**

(74) Representative: **Murgitroyd & Company
Murgitroyd House
165-169 Scotland Street
Glasgow G5 8PL (GB)**

(54)  **COMPOSITION OF GINSENOSIDES RG3 AND RG5 AND ANTI-TUMOR PHARMACEUTICAL USE THEREOF**

(57)    The present invention discloses a composition of ginsenoside Rg3 and ginsenoside Rg5 and a preparation method thereof, as well as its application in manufacturing drugs, foods and health products for boosting immunity, enhancing anti-tumor effects, improving resistance to anti-tumor targeted drugs, mitigating toxic and side effects of radiotherapy and chemotherapy or improving anti-fatigue effects. The composition has advantages in rapid action, less toxicity and side effects and is suitable for long-term use. It is a kind of safe, highly efficient and stable drug, food and health products that requires simple preparation process, is suitable for industrial production and can be easily popularized. The present invention provides a new type of raw material and production process for manufacturing drugs, foods and health products for boosting immunity, enhancing anti-tumor effect, improving resistance to anti-tumor targeted drugs, mitigating toxic and side effects of radiotherapy and chemotherapy or improving anti-fatigue effect.

EP 4 205 748 A1

## Description

## Technical Field

The present invention belongs to the field of medicinal chemistry. To be specific, the invention involves a composition of ginsenoside Rg3 and ginsenoside Rg5 and a preparation method thereof, as well as its application in manufacturing drugs, foods and health products for boosting immunity, enhancing anti-tumor effect, improving resistance to anti-tumor targeted drugs, mitigating toxic and side effects of radiotherapy and chemotherapy or improving anti-fatigue effect.

## Background Technologies

Panax ginseng belongs to the genus Panax of the Araliaceae family. It has been widely used in China, Korea and Japan for more than 2000 years. Its main purpose is to prevent diseases and prolong life expectancy. According to the records in the Shennong's Classic of Materia Medica, panax ginseng has the therapeutic effects of nourishing the five viscera, calming the nerves, settling the mind, stopping palpitation due to fright, eliminating evil, improving the eyesight and intelligence, conducive to controlling body weight and prolonging life expectancy. Modern medical studies have shown that panax ginseng's main functions and effects include regulating the central nervous system, exerting anti-cancer and anti-tumor effects, regulating immune functions, exerting anti-diabetic effects, enhancing liver function, improving cardiovascular and cerebrovascular disorders, exerting anti-arteriosclerosis effects, regulating blood pressure, improving climacteric disorders, exerting anti-osteoporosis effects, and exerting anti-fatigue, anti-oxidation as well as anti-aging effects, etc.

As the main effective components of panax ginseng, ginsenoside Rg3 and ginsenoside Rg5 have good safety profiles and have been developed as the anti-tumor oral preparations for clinical application and in-depth studies have been conducted for their dosage forms of injection.

However, the present inventors have found out that, in the prior art, the pharmacological effects of panax ginseng are either investigated by testing one or several extracted and purified ginsenosides, in which the amounts of ginsenoside Rg3 and ginsenoside Rg5 are often relatively close, or by testing the mixtures or extracts of a large number of different kinds of Chinese medicinal materials (including panax ginseng) are studied, in which the contents of ginsenoside Rg3 and ginsenoside Rg5 are usually not determined. Based on the long-term research experience in traditional Chinese medicine and modern medicine, the inventors have developed a method for preparing a composition of ginsenoside Rg3 and ginsenoside Rg5, which is not only simple, requiring mild process conditions, but also has multiple formulas for wide applications. A large number of effective compositions of ginsenoside Rg3 and ginsenoside Rg5 have been obtained, which can be widely used in boosting immunity, exerting anti-tumor effects, improving resistance to anti-tumor drugs, reducing toxic and side effects caused by radiotherapy and chemotherapy, and exerting anti-fatigue effects, etc., and has broad prospects in application in the fields of drug preparation, foods and health products, etc.

## Description of the Present Invention

The composition of ginsenoside Rg3 and ginsenoside Rg5 of the present invention boasts strong efficacy in boosting immunity, exerting anti-tumor effects, improving the resistance to anti-tumor drugs, reducing toxic and side effects caused by radiotherapy and chemotherapy, and exerting anti-fatigue effects. It also provides a new approach to developing new drugs to treat these diseases. The present invention has shed new lights on developing novel applications in drugs, foods or health products for treating, regulating and relieving such diseases.

To achieve the above goals, the present invention provides a composition of ginsenoside Rg3 and ginsenoside Rg5, the related preparation method and the application thereof.

In particular, the said ginsenoside Rg3 is one with the S-type conformation, or that with the R type conformation, or a mixture of these two types of ginsenoside Rg3 in any ratio.

In particular, in the said composition of ginsenoside Rg3 and ginsenoside Rg5, the weight ratio of ginsenoside Rg3 to ginsenoside Rg5 is 1-99: 1-99.

In particular, the said composition of ginsenoside Rg3 and ginsenoside Rg5 is a mixture of panax ginseng and other Chinese medicinal materials prepared using a specific process, and the percentage of the weight of ginsenoside Rg3 to ginsenoside Rg5 in the mixture of Chinese medicinal components is 1% to 100%.

In particular, the said composition of ginsenoside Rg3 and ginsenoside Rg5 also includes a complex of a mixture of panax ginseng and other Chinese medicinal materials prepared using a specific process and pharmaceutically acceptable carriers processed and formed using both physical and chemical methods.

In particular, for the pharmaceutically acceptable carriers, including but not limited to α-, β- or γ-cyclodextrin or the derivatives thereof, the weight ratio of the mixture of Chinese medicinal components to the carrier is 1:1 to 1:100, preferably in 1:10, further preferably in 1: 5, still further preferably in 1:2, and still further preferably in 1:1.

In particular, pharmaceutically acceptable carriers are generally accepted by health professionals as suitable for this purpose and as inactive ingredients in pharmaceutical agents. The compilation of pharmaceutically acceptable carriers can be found in the Handbook of Pharmaceutical Excipients, 2nd edition, edited by A. Wade and P. J. Weller, and published by the American Pharmaceutical Association, Washington and The Pharmaceutical Press, London, 1994. Food additives are non-nutritive substances that are consciously added to foods in small amounts to improve the appearance, flavor, texture or storage properties of foods. Relevant food additives can be found in China's national food safety standard and the Standards for Use of Food Additives. In particular, the said carrier comprises excipients such as starch and water, lubricants such as magnesium stearate, disintegrants such as microcrystalline cellulose, filling agents such as lactose; binding agents such as pregelatinized starch and dextrin, sweeteners, antioxidants, preservatives, flavoring agents, colorants and spices.

In particular, the said drug may exist in the dosage forms of tablets, capsules, pills, powder, granules, syrup, solution, emulsion, injection, spray, aerosol, gel, cream, cataplasm, adhesive plaster or emplastrum.

In particular, the said foodstuffs may exist in the forms of foods such as dairy products, confectionery, beverages, biscuits, tea leaves and related products, wine and the like.

In particular, the said health food products may exist in the forms of tablets, capsules, pills, powder, granules, syrup, solution, emulsion, spray, aerosol, gel, cream, cataplasm, adhesive plaster or emplastrums, or in the forms of foods such as dairy products, confectionery, beverages, biscuits, tea leaves and related products, wine and the like.

Wherein the weight ratio of the traditional Chinese medicine components to the cyclodextrins or the derivatives thereof in the said complex is 1:1-100.

In particular, the said cyclodextrin is α-, β- or γ-cyclodextrin; the said cyclodextrin derivatives are hydroxyethyl□-cyclodextrin, 2,6-dimethyl□-cyclodextrin, 2,3,6-trimethyl□-cyclodextrin, 2,6-diethyl□-cyclodextrin, 2,3,6-triethyl□-cyclodextrin, maltosyl□-cyclodextrin or sulfobutylether β-cyclodextrin, p-toluenesulfonyl chloride (p-TsCl) substituted β-cyclodextrin, 6-position substituted β-CD p-toluenesulfonate (β-cyclodextrin-6-OTs), 2-oxohydroxypropyl-β-cyclodextrin, 2-position monosubstituted p-toluenesulfonate (2-β-cyclodextrin-2-OTs), β-cyclodextrin p-toluenesulfonate (tosyl-β-CD) and PCL-(Tos) 7-β-CD, the star-shaped macromolecule of β-cyclodextrin.

More particularly, the first aspect of the present invention provides a method for preparing the composition containing ginsenoside Rg3 and ginsenoside Rg5, which comprises the following steps:

1) Mix panax ginseng with Chinese medicinal materials;
2) Decoct the mixture obtained from step 1) with water several times, and collect and combine the decoction fluid;
3) Add a clarifying agent to the decoction fluid obtained from step 2), mix well, allow to stand and filter;
4) Dry the filtrate obtained from step 3) to obtain the dried substance;
5) Add ethanol aqueous solution to the dried substance obtained from step 4), heat to dissolve, filter, and subject to the low temperature crystallization process;
6) Dry the crystals obtained from step 5).

Wherein, the said Chinese medicinal material is not panax ginseng. Preferably, in the method of the first aspect of the invention, the said Chinese medicinal materials are selected from the groups of one or more of the following medicines, including mulberry, fried aurantii fructus, paeoniae radix alba, citri sarcodactylis fructus, nelumbinis rhizomatis nodus, fried aurantii fructus immaturus, gingko nut, diospyros kaki calyx, aconitum kusnezoffii leaf, lotus seedpod, ailanthi cortex, Chinese arborvitae twig, punica granatum peel, comus officinalis, gallnut, rhizoma bletillae, white aconite, fructus toosendan, trichosanthis radix, codonopsis pilosula, purple iris, artemisia annua, chinese honeylocust spine, fructus foeniculi, kochia scoparia fruit, peach kernel, mume fructus, herba epimedii, chicken's gizzard-membrane, root of eichmannia, dioscorea oppositifolia, gentiana macrophylla root, adenophora stricta root, pyrrosia lingua, salvia miltiorrhiza, pharbitidis semen, rigonellae semen, lablab purpureus seed, fructus perillae, rhizoma pinelliae preparata, coptis chinensis rhizome, cullen corylifolium fruit, phellodendri chinensis cortex, rhizoma corydalis, canarii fructus, eclipta prostrata, papaya, portulacae herba, citrus medica, achyranthes bidentata, rubus chingii, fallopia multiflora, fraxini cortex, trachycarpus fortunei, nelumbinis semen, agrimonia pilosa herb, green plum, alumen, papaveris pericarpium, terminalia chebula, rosa laevigata, sanguisorba officinalis, prunus dulcis, citrus reticulata pericarp, gardenia jasminoides, ligustri lucidi fructus, rehmannia glutinosa processed root tuber, scutellariae radix, reed rhizome, astragali radix, curcumae rhizoma, mori cortex, ophiopogonis radix, arecae semen, raw frankincense, spatholobi caulis, atractylodis macrocephalae rhizoma, lycii cortex, angelica sinensis, prunus mume smoke treated unripe fruit, hordeum vulgare sprout, eriobotrya japonica leaf, platycladus orientalis seed, cinnamon, asparagi radix, raw myrrh, platycodonis radix, perotis indica, processed radix aconiti lateralis, chrysanthemi flos, aucklandiae radix, radix stemonae, sparganii rhizoma, eucommiae cortex, citrus reticulata fruit peel, rheum officinale, pericarpium arecae, gastrodia elata, pseudostellariae radix, paeonia rubra root, flos inulae, prunella vulgaris, cuscuta chinensis seed, drynaria fortunei, curcumae radix, arisaema cum bile, radix stemonae, taraxacum mongolicum herb, licorice, cimicifugae rhizoma, corydalis bungeana, rubia cordifolia, linderae radix, acanthopanax gracilistylus root bark, aconiti radix, lichee exocarp, emblic leafflower fruit, belamcandae rhizoma, folium isatidis, euodiae fructus, carthami flos, cyperi rhizoma, schisandra chinensis, ziziphus jujuba seed, crataegi fructus and radix lithospermi.

More preferably, the said Chinese medicinal materials are selected from the groups of one or more of the following medicines, including curcumae radix, arisaema cum bile, radix stemonae, chrysanthemi flos, aucklandiae radix, schisandra chinensis, sparganii rhizoma, atractylodis macrocephalae rhizoma, fructus phyllanthi fruit, pharbitidis semen, phellodendri chinensis cortex, cullen corylifolium fruit, lablab purpureus seed, prunus mume smoke treated unripe fruit, fructus perillae, salvia miltiorrhiza, pyrrosia lingua, dioscorea oppositifolia, root of eichmannia, gentiana macrophylla root, adenophora stricta root, coptis chinensis rhizome, rigonellae semen, rhizoma corydalis, green plum, eucommiae cortex, citrus reticulata fruit peel, ziziphus jujuba seed, gingko nut, punica granatum peel, peach kernel, rhizoma bletillae, cuscuta chinensis seed, crataegi fructus, paeoniae radix alba, Chinese arborvitae twig, pseudostellariae radix, paeonia rubra root, fructus foeniculi, artemisia annua, gallnut, trichosanthis radix, codonopsis pilosula, fructus toosendan, comus officinalis, aurantii fructus immaturus, lotus seedpod, mulberry, chicken's gizzard-membrane, aurantii fructus, ailanthi cortex, aconitum kusnezoffii, michaelmas daisy, chinese honeylocust spine, kochia scoparia fruit, pinelliae rhizoma, rhei radix et rhizoma, gastrodia elata, flos inulae and prunella vulgaris.

Preferably, in the method of the first aspect of the present invention, the weight ratio of panax ginseng to the Chinese medicinal materials is 1:100 to 100:1, for example, 1: 90-100, 1:45-55, 10: 8-12, 45-55:1 or 90-100:1, preferably in 1:100, 1:50, 10:10, 50:1 or 100:1.

Preferably, in the method of the first aspect of the present invention, the number of decoctions is 2-5 times, preferably 3 times. Each time after the completion of decoction, collect the decoction liquid for later use. If more decoction is required by adding water, take the filter residue after the collection of the decoction liquid and add water again to decoct until the last decoction by adding water is completed and then combine the obtained liquid with the previously collected decoction liquids.

Preferably, in the method of the first aspect of the present invention, the weight of water used for each decoction is 2-100 times, preferably 2-80 times and more preferably 2-50 times, the total weight of panax ginseng and the Chinese medicinal materials.

Preferably, in the method of the first aspect of the present invention, each decoction time is 1-36 h, preferably 2-24 h and more preferably 5-12 h.

Preferably, in the method of the first aspect of the present invention, the temperature for each decoction is 90-100°C, preferably 95-100°C, for example, decoct to maintain the boiling state. Decoction is carried out in a tightly sealed container.

Preferably, in the method of the first aspect of the present invention, the clarifying agent is an acceptable clarifying agent in the industries of pharmaceutics and/or foodstuff, such as a natural clarifying agent. Such clarifiers are commercially available, such as ZTC1+1 clarifiers. The added amount of a ZTC1+1 clarifier is 200-800 ppm, i.e., the final concentration of the reagent.

Preferably, in the method of the first aspect of the present invention, the standing time is 4-48 h, preferably, 4-24 h and more preferably 8-12 h.

Preferably, in the method of the first aspect of the present invention, the concentration of the aqueous ethanol solution used for crystallization is 40-90% (V/V), preferably 50-80% (V/V). More preferably, the weight of the aqueous solution of ethanol is 2-500 times, and further preferably 10-100 times, the weight of the dried substance obtained by drying the filtrate obtained in step 3).

Preferably, in the method of the first aspect of the invention, a detection step following step 6) may also be included. For example, the content a component such as ginsenoside Rg3 and/or ginsenoside Rg5 is determined by the high performance liquid chromatography method.

A second aspect of the present invention provides a composition containing ginsenoside Rg3 and ginsenoside Rg5, which is prepared by the method of the first aspect of the present invention. Wherein, ginsenoside Rg3 can be S-type ginsenoside Rg3, or R-type ginsenoside Rg3, or a mixture of the above two conformations. Preferably, in the composition of the second aspect of the present invention, the weight ratio of ginsenoside Rg3 to ginsenoside Rg5 is 1-99:1-99.

Preferably, in the composition of the second aspect of the present invention, the percentage of the total weight ginsenoside Rg3 and ginsenoside Rg5 in the composition is 1% to 100%, preferably 2% to 99%, such as 98.0% to 100% or 98.0% to 99%.

Preferably, in the composition of the second aspect of the present invention, the weight ratio of ginsenoside Rg3 to ginsenoside Rg5 is 1:99 to 99:1.

The third aspect of the present invention provides a complex composite that includes the composition of the second aspect of the invention and an acceptable carrier in the industries of pharmaceutics or foods. Preferably, in the complex composition of the third aspect of the present invention, the weight ratio of the composition of the second aspect of the present invention to the acceptable carrier in the industries of pharmaceutics or foods is 1:1 to 1:100.

Preferably, the complex composition of the third aspect of the present invention may be a pharmaceutical composition that includes the composition of the second aspect of the present invention and a pharmaceutically acceptable carrier. A pharmaceutically acceptable carrier including, but not limited to, $\alpha$-, $\beta$- or $\gamma$-cyclodextrin or the derivatives thereof. Preferably, the said pharmaceutical composition may exist in the dosage forms of tablets, capsules, pills, powder, granules, syrup, solution, emulsion, injection, spray, aerosol, gel, cream, cataplasm, adhesive plaster or emplastrum.

Preferably, the complex composition of the third aspect of the present invention may be a food composition, which may exist in the forms of foods such as dairy products, confectionery, beverages, biscuits, tea leaves and related products, wine and the like.

Preferably, the complex composition of the third aspect of the present invention may be a composition of health products, which may exist in the forms of tablets, capsules, pills, powder, granules, syrup, solution, emulsion, spray, aerosol, gel, cream, cataplasm, adhesive plaster or emplastrums, or in the forms of foods such as dairy products, confectionery, beverages, biscuits, tea leaves and related products, wine and the like.

The fourth aspect of the present invention provides the application of the composition of the second aspect of the present invention in manufacturing drugs, foods and health products for boosting immunity, enhancing anti-tumor effects, improving resistance to anti-tumor targeted drugs, mitigating toxic and side effects of radiotherapy and chemotherapy or improving anti-fatigue effect. Accordingly, the fifth aspect of the present invention provides a method for boosting immunity, enhancing anti-tumor effect, improving resistance to anti-tumor targeted drugs, mitigating toxic and side effects caused by radiotherapy and chemotherapy or improving anti-fatigue effect, including the administration of the composition of the second aspect of the present invention at an effective amount to individuals who need the composition.

Wherein, preferably, the individuals are humans, and the effective dose for humans can be calculated by converting the effective dose obtained from animal experiments to the equivalent dose for humans.

Compared to the prior art, the present invention has the following obvious advantages:

The preparation method of the composition of ginsenoside Rg3 and ginsenoside Rg5 of the present invention is simple and suitable for industrial production. The said composition has obvious effects of boosting immunity, enhancing anti-tumor effect, improving resistance to anti-tumor targeted drugs, mitigating toxic and side effects caused by radiotherapy and chemotherapy or improving anti-fatigue effect, and has good prospects for application.

1. The composition of ginsenoside Rg3 and ginsenoside Rg5 of the invention has a unique formula, high safety profile and controllable quality;

2. The composition of the present invention explores new pharmaceutical values, and a series of experimental studies have proved that the composition has the effects of boosting immunity, enhancing anti-tumor effect, improving resistance to anti-tumor targeted drugs, mitigating toxic and side effects caused by radiotherapy and chemotherapy or improving anti-fatigue effect, with definite curative effect;

3. The composition of the present invention has the following advantages: it is simple to prepare and requires mild process conditions, the cost and price are low, and it has multiple formulas for wide applications;

4. The product of the present invention has an abundant source of raw materials, a low price, safe clinical uses, a simple preparation process, and can be prepared into various dosage forms; moreover, the dosage is small and the use is convenient. Hence, it is easy to be popularized.

**Detailed Embodiment of the Invention**

The beneficial effects of the formulations described in the present invention are further described below using the specific embodiments. These exemplary embodiments are exemplary only and do not constitute any limitation to the scope of the present invention. It should be appreciated by those skilled in the art that the details and forms of the technical proposal of the present invention can be modified or substituted without departing from the formulation ideas and scope of use of the present invention. However, all these modifications and substitutions fall within the scope of protection of the present invention.

**Exemplary Embodiments 1-180: Preparation of a Composition of Ginsenoside Rg3 and Ginsenoside Rg5**

The specific process route is as follows: decoct the mixture of panax ginseng and other raw materials with water three times, pool the water solution and concentrate the mixture to the concentration of crude drug/water of 1: 5 (v/v), maintain the temperature at 60-80°C, add ZTC1+1-II clarifying agent (it can be purchased from Wuhan Zheng Tian Cheng Biological Science & Technology Co., Ltd.) to its final concentration of 500 ppm, stir evenly, allow to stand, filter, dry the filtrate, add ethanol solution to dissolve at 60°C, cool after filtration, separate the crystals in an ice water bath, collect the precipitate, and dry to obtain the finished product. The content of each component is determined by HPLC.

The panax ginseng is respectively weighed with various crude drugs according to the weights described in Table 1 and then mixed and extracted in accordance with the above process route and the parameters described in Table 2 for preparation. The test data of the obtained finished product are presented in Table 3.

**Table 1 Raw Material Ratio and Finished Product Weight of Exemplary Embodiment 1-180**

| Composition Serial No. | Other raw materials | Weight of panax ginseng (g) | Weight of other raw materials (g) | Amount of finished products (g) |
|---|---|---|---|---|
| 1 (or A) | Curcumae radix | 100 | 1 | 3.2 |
| 2 (or B) | | 100 | 10000 | 23.6 |
| 3 (or C) | | 100 | 5000 | 8.1 |
| 4 (or D) | Arisaema cum bile | 100 | 100 | 6.2 |
| 5 (or E) | | 100 | 10000 | 22.0 |
| 6 (or F) | | 100 | 5000 | 7.9 |
| 7 (or G) | Radix stemonae | 100 | 1 | 3.1 |
| 8 (or H) | | 100 | 10000 | 22.5 |
| 9 (or I) | | 100 | 5000 | 7.8 |
| 10 | Chrysanthemi flos | 100 | 1 | 2.8 |
| 11 | | 100 | 10000 | 19.3 |
| 12 | | 100 | 5000 | 6.4 |
| 13 | Aucklandiae radix | 100 | 100 | 6.0 |
| 14 | | 100 | 10000 | 21.8 |
| 15 | | 100 | 5000 | 7.7 |
| 16 | Schisandra chinensis | 100 | 1 | 3.3 |
| 17 | | 100 | 10000 | 22.9 |
| 18 | | 100 | 5000 | 7.9 |
| 19 | Sparganii rhizoma | 100 | 1 | 3.7 |
| 20 | | 100 | 10000 | 22.9 |
| 21 | | 100 | 5000 | 8.5 |
| 22 | Atractylodis macrocephalae rhizoma | 100 | 100 | 6.6 |
| 23 | | 100 | 10000 | 21.9 |
| 24 | | 100 | 5000 | 7.7 |
| 25 | Phyllanthus emblica | 100 | 1 | 3.3 |
| 26 | | 100 | 10000 | 22.5 |
| 27 | | 100 | 5000 | 7.9 |
| 28 | Pharbitidis Semen | 100 | 1 | 3.0 |
| 29 | | 100 | 10000 | 23.1 |
| 30 | | 100 | 5000 | 8.0 |
| 31 | Phellodendri chinensis cortex | 100 | 100 | 6.4 |
| 32 | | 100 | 10000 | 23.4 |
| 33 | | 100 | 5000 | 8.2 |

(continued)

| Composition Serial No. | Other raw materials | Weight of panax ginseng (g) | Weight of other raw materials (g) | Amount of finished products (g) |
|---|---|---|---|---|
| 34 | Cullen corylifolium fruit | 100 | 1 | 3.2 |
| 35 | | 100 | 10000 | 22.8 |
| 36 | | 100 | 5000 | 8.3 |
| 37 | Lablab purpureus seed | 100 | 1 | 2.9 |
| 38 | | 100 | 10000 | 23.3 |
| 39 | | 100 | 5000 | 7.7 |
| 40 | Prunus mume smoke treated unripe fruit | 100 | 100 | 6.9 |
| 41 | | 100 | 10000 | 22.8 |
| 42 | | 100 | 5000 | 8.3 |
| 43 | Fructus perillae | 100 | 1 | 3.8 |
| 44 | | 100 | 10000 | 22.8 |
| 45 | | 100 | 5000 | 8.2 |
| 46 | Salvia miltiorrhiza | 100 | 1 | 3.8 |
| 47 | | 100 | 10000 | 24.0 |
| 48 | | 100 | 5000 | 9.3 |
| 49 | Pyrrosia lingua | 100 | 100 | 7.4 |
| 50 | | 100 | 10000 | 22.0 |
| 51 | | 100 | 5000 | 7.9 |
| 52 | Dioscorea oppositifolia | 100 | 1 | 4.5 |
| 53 | | 100 | 10000 | 24.9 |
| 54 | | 100 | 5000 | 8.8 |
| 55 | Dried rhizome of rehmannia | 100 | 1 | 4.7 |
| 56 | | 100 | 10000 | 26.7 |
| 57 | | 100 | 5000 | 10.3 |
| 58 | Gentiana macrophylla root | 100 | 100 | 6.1 |
| 59 | | 100 | 10000 | 21.8 |
| 60 | | 100 | 5000 | 8.3 |
| 61 | Adenophora stricta root | 100 | 1 | 4.2 |
| 62 | | 100 | 10000 | 25.6 |
| 63 | | 100 | 5000 | 8.7 |
| 64 | Coptis chinensis rhizome | 100 | 1 | 3.7 |
| 65 | | 100 | 10000 | 25.0 |
| 66 | | 100 | 5000 | 9.3 |
| 67 | Faenum | 100 | 100 | 5.3 |
| 68 | graecum | 100 | 10000 | 18.6 |
| 69 | | 100 | 5000 | 4.6 |

(continued)

| Composition Serial No. | Other raw materials | Weight of panax ginseng (g) | Weight of other raw materials (g) | Amount of finished products (g) |
|---|---|---|---|---|
| 70 | Rhizoma corydalis | 100 | 1 | 3.9 |
| 71 | Rhizoma corydalis | 100 | 10000 | 24.8 |
| 72 | Rhizoma corydalis | 100 | 5000 | 9.0 |
| 73 | Prunus mume | 100 | 1 | 3.6 |
| 74 | Prunus mume | 100 | 10000 | 22.8 |
| 75 | Prunus mume | 100 | 5000 | 7.3 |
| 76 | Eucommiae cortex | 100 | 100 | 6.4 |
| 77 | Eucommiae cortex | 100 | 10000 | 21.2 |
| 78 | Eucommiae cortex | 100 | 5000 | 8.9 |
| 79 | Citrus reticulata fruit peel | 100 | 1 | 4.2 |
| 80 | Citrus reticulata fruit peel | 100 | 10000 | 23.6 |
| 81 | Citrus reticulata fruit peel | 100 | 5000 | 8.9 |
| 82 | Ziziphus jujuba seed | 100 | 1 | 4.5 |
| 83 | Ziziphus jujuba seed | 100 | 10000 | 25.9 |
| 84 | Ziziphus jujuba seed | 100 | 5000 | 7.4 |
| 85 | Ginkgo nut | 100 | 100 | 6.7 |
| 86 | Ginkgo nut | 100 | 10000 | 21.5 |
| 87 | Ginkgo nut | 100 | 5000 | 6.4 |
| 88 | Punic a granatum | 100 | 1 | 1.7 |
| 89 | Punic a granatum | 100 | 10000 | 23.1 |
| 90 | Punic a granatum | 100 | 5000 | 8.4 |
| 91 | Peach kernel | 100 | 1 | 4.9 |
| 92 | Peach kernel | 100 | 10000 | 23.3 |
| 93 | Peach kernel | 100 | 5000 | 8.8 |
| 94 | Bletilla striata | 100 | 100 | 6.0 |
| 95 | Bletilla striata | 100 | 10000 | 22.8 |
| 96 | Bletilla striata | 100 | 5000 | 7.7 |
| 97 | Cuscuta chinensis seed | 100 | 1 | 3.0 |
| 98 | Cuscuta chinensis seed | 100 | 10000 | 22.4 |
| 99 | Cuscuta chinensis seed | 100 | 5000 | 7.7 |
| 100 | Crataegus pinnatifida | 100 | 1 | 3.2 |
| 101 | Crataegus pinnatifida | 100 | 10000 | 23.1 |
| 102 | Crataegus pinnatifida | 100 | 5000 | 8.2 |
| 103 | Paeoniae radix alba | 100 | 100 | 6.3 |
| 104 | Paeoniae radix alba | 100 | 10000 | 22.9 |
| 105 | Paeoniae radix alba | 100 | 5000 | 7.0 |

(continued)

| Composition Serial No. | Other raw materials | Weight of panax ginseng (g) | Weight of other raw materials (g) | Amount of finished products (g) |
|---|---|---|---|---|
| 106 | Platycladus orientalis leaf | 100 | 1 | 3.0 |
| 107 | | 100 | 10000 | 22.7 |
| 108 | | 100 | 5000 | 7.6 |
| 109 | Pseudostellaria heterophylla | 100 | 1 | 3.4 |
| 110 | | 100 | 10000 | 23.4 |
| 111 | | 100 | 5000 | 8.2 |
| 112 | Paeonia rubra root | 100 | 100 | 6.9 |
| 113 | | 100 | 10000 | 22.4 |
| 114 | | 100 | 5000 | 7.5 |
| 115 | Foeniculum vulgare dry fruit | 100 | 1 | 3.6 |
| 116 | | 100 | 10000 | 22.0 |
| 117 | | 100 | 5000 | 7.4 |
| 118 | Artemisia annua | 100 | 1 | 3.2 |
| 119 | | 100 | 10000 | 23.3 |
| 120 | | 100 | 5000 | 8.4 |
| 121 | Rhus spp. galls | 100 | 100 | 6.0 |
| 122 | | 100 | 10000 | 22.2 |
| 123 | | 100 | 5000 | 7.8 |
| 124 | Trichosanthes spp. root | 100 | 1 | 3.2 |
| 125 | | 100 | 10000 | 22.5 |
| 126 | | 100 | 5000 | 7.7 |
| 127 | Codonopsis pilosula | 100 | 1 | 3.3 |
| 128 | | 100 | 10000 | 23.9 |
| 129 | | 100 | 5000 | 8.8 |
| 130 | Toosendan fructus | 100 | 100 | 6.6 |
| 131 | | 100 | 10000 | 22.6 |
| 132 | | 100 | 5000 | 7.4 |
| 133 | Corni fructus | 100 | 1 | 3.6 |
| 134 | | 100 | 10000 | 22.1 |
| 135 | | 100 | 5000 | 7.2 |
| 136 | Citrus aurantium | 100 | 1 | 3.9 |
| 137 | | 100 | 10000 | 23.9 |
| 138 | | 100 | 5000 | 8.1 |
| 139 | Lotus seedpod | 100 | 100 | 6.1 |
| 140 | | 100 | 10000 | 22.1 |
| 141 | | 100 | 5000 | 7.0 |

(continued)

| Composition Serial No. | Other raw materials | Weight of panax ginseng (g) | Weight of other raw materials (g) | Amount of finished products (g) |
|---|---|---|---|---|
| 142 | Mulberry | 100 | 1 | 3.3 |
| 143 | | 100 | 10000 | 22.7 |
| 144 | | 100 | 5000 | 7.1 |
| 145 | Chicken's gizzard-membra ne | 100 | 1 | 3.5 |
| 146 | | 100 | 10000 | 23.0 |
| 147 | | 100 | 5000 | 8.5 |
| 148 | Aurantii Fructus | 100 | 100 | 6.7 |
| 149 | | 100 | 10000 | 22.5 |
| 150 | | 100 | 5000 | 7.5 |
| 151 | Ailanthi cortex | 100 | 1 | 3.2 |
| 152 | | 100 | 10000 | 22.8 |
| 153 | | 100 | 5000 | 7.3 |
| 154 | Aconitum kusnezoffii | 100 | 1 | 3.9 |
| 155 | | 100 | 10000 | 23.5 |
| 156 | | 100 | 5000 | 8.3 |
| 157 | Michaelmas daisy | 100 | 100 | 6.4 |
| 158 | | 100 | 10000 | 22.6 |
| 159 | | 100 | 5000 | 7.7 |
| 160 | Chinese honeylocust spine | 100 | 1 | 3.1 |
| 161 | | 100 | 10000 | 22.6 |
| 162 | | 100 | 5000 | 7.1 |
| 163 | Kochia scoparia fruit | 100 | 1 | 3.7 |
| 164 | | 100 | 10000 | 23.3 |
| 165 | | 100 | 5000 | 8.0 |
| 166 | Pinellia ternata | 100 | 100 | 6.4 |
| 167 | | 100 | 10000 | 22.5 |
| 168 | | 100 | 5000 | 7.5 |
| 169 | Rheum officinale | 100 | 1 | 3.9 |
| 170 | | 100 | 10000 | 22.5 |
| 171 | | 100 | 5000 | 7.9 |
| 172 | Gastrodia elata | 100 | 1 | 3.1 |
| 173 | | 100 | 10000 | 23.8 |
| 174 | | 100 | 5000 | 8.0 |
| 175 | Flos inulae | 100 | 100 | 6.5 |
| 176 | | 100 | 10000 | 22.9 |
| 177 | | 100 | 5000 | 7.4 |

(continued)

| Composition Serial No. | Other raw materials | Weight of panax ginseng (g) | Weight of other raw materials (g) | Amount of finished products (g) |
|---|---|---|---|---|
| 178 | | 100 | 1 | 3.7 |
| 179 | Prunella vulgaris | 100 | 10000 | 22.2 |
| 180 | | 100 | 5000 | 7.0 |

**Table 2 Sample Preparation Process Parameters for Exemplary Embodiment 1-180**

| Composition Serial No. | Amount of water (ml) | Standing time (h) | Content of ethanol (%) | Amount of ethanol (ml) |
|---|---|---|---|---|
| 1 | 4000 | 8 | 50 | 200 |
| 2 | 400000 | 8 | 50 | 2000 |
| 3 | 250000 | 8 | 60 | 1000 |
| 4 | 8000 | 12 | 60 | 200 |
| 5 | 400000 | 12 | 70 | 2000 |
| 6 | 200000 | 12 | 70 | 1000 |
| 7 | 5000 | 24 | 80 | 200 |
| 8 | 500000 | 24 | 80 | 2000 |
| 9 | 250000 | 24 | 80 | 1000 |
| 10 | 4000 | 8 | 50 | 200 |
| 11 | 400000 | 8 | 50 | 2000 |
| 12 | 250000 | 8 | 60 | 1000 |
| 13 | 8000 | 12 | 60 | 200 |
| 14 | 400000 | 12 | 70 | 2000 |
| 15 | 200000 | 12 | 70 | 1000 |
| 16 | 5000 | 24 | 80 | 200 |
| 17 | 500000 | 24 | 80 | 2000 |
| 18 | 250000 | 24 | 80 | 1000 |
| 19 | 4000 | 8 | 50 | 200 |
| 20 | 400000 | 8 | 50 | 2000 |
| 21 | 250000 | 8 | 60 | 1000 |
| 22 | 8000 | 12 | 60 | 200 |
| 23 | 400000 | 12 | 70 | 2000 |
| 24 | 200000 | 12 | 70 | 1000 |
| 25 | 5000 | 24 | 80 | 200 |
| 26 | 500000 | 24 | 80 | 2000 |
| 27 | 250000 | 24 | 80 | 1000 |
| 28 | 4000 | 8 | 50 | 200 |
| 29 | 400000 | 8 | 50 | 2000 |
| 30 | 250000 | 8 | 60 | 1000 |

(continued)

| Composition Serial No. | Amount of water (ml) | Standing time (h) | Content of ethanol (%) | Amount of ethanol (ml) |
|---|---|---|---|---|
| 31 | 8000 | 12 | 60 | 200 |
| 32 | 400000 | 12 | 70 | 2000 |
| 33 | 200000 | 12 | 70 | 1000 |
| 34 | 5000 | 24 | 80 | 200 |
| 35 | 500000 | 24 | 80 | 2000 |
| 36 | 250000 | 24 | 80 | 1000 |
| 37 | 4000 | 8 | 50 | 200 |
| 38 | 400000 | 8 | 50 | 2000 |
| 39 | 250000 | 8 | 60 | 1000 |
| 40 | 8000 | 12 | 60 | 200 |
| 41 | 400000 | 12 | 70 | 2000 |
| 42 | 200000 | 12 | 70 | 1000 |
| 43 | 5000 | 24 | 80 | 200 |
| 44 | 500000 | 24 | 80 | 2000 |
| 45 | 250000 | 24 | 80 | 1000 |
| 46 | 4000 | 8 | 50 | 200 |
| 47 | 400000 | 8 | 50 | 2000 |
| 48 | 250000 | 8 | 60 | 1000 |
| 49 | 8000 | 12 | 60 | 200 |
| 50 | 400000 | 12 | 70 | 2000 |
| 51 | 200000 | 12 | 70 | 1000 |
| 52 | 5000 | 24 | 80 | 200 |
| 53 | 500000 | 24 | 80 | 2000 |
| 54 | 250000 | 24 | 80 | 1000 |
| 55 | 4000 | 8 | 50 | 200 |
| 56 | 400000 | 8 | 50 | 2000 |
| 57 | 250000 | 8 | 60 | 1000 |
| 58 | 8000 | 12 | 60 | 200 |
| 59 | 400000 | 12 | 70 | 2000 |
| 60 | 200000 | 12 | 70 | 1000 |
| 61 | 5000 | 24 | 80 | 200 |
| 62 | 500000 | 24 | 80 | 2000 |
| 63 | 250000 | 24 | 80 | 1000 |
| 64 | 4000 | 8 | 50 | 200 |
| 65 | 400000 | 8 | 50 | 2000 |
| 66 | 250000 | 8 | 60 | 1000 |
| 67 | 8000 | 12 | 60 | 200 |
| 68 | 400000 | 12 | 70 | 2000 |

(continued)

| Composition Serial No. | Amount of water (ml) | Standing time (h) | Content of ethanol (%) | Amount of ethanol (ml) |
|---|---|---|---|---|
| 69 | 200000 | 12 | 70 | 1000 |
| 70 | 5000 | 24 | 80 | 200 |
| 71 | 500000 | 24 | 80 | 2000 |
| 72 | 250000 | 24 | 80 | 1000 |
| 73 | 4000 | 8 | 50 | 200 |
| 74 | 400000 | 8 | 50 | 2000 |
| 75 | 250000 | 8 | 60 | 1000 |
| 76 | 8000 | 12 | 60 | 200 |
| 77 | 400000 | 12 | 70 | 2000 |
| 78 | 200000 | 12 | 70 | 1000 |
| 79 | 5000 | 24 | 80 | 200 |
| 80 | 500000 | 24 | 80 | 2000 |
| 81 | 250000 | 24 | 80 | 1000 |
| 82 | 4000 | 8 | 50 | 200 |
| 83 | 400000 | 8 | 50 | 2000 |
| 84 | 250000 | 8 | 60 | 1000 |
| 85 | 8000 | 12 | 60 | 200 |
| 86 | 400000 | 12 | 70 | 2000 |
| 87 | 200000 | 12 | 70 | 1000 |
| 88 | 5000 | 24 | 80 | 200 |
| 89 | 500000 | 24 | 80 | 2000 |
| 90 | 250000 | 24 | 80 | 1000 |
| 91 | 4000 | 8 | 50 | 200 |
| 92 | 400000 | 8 | 50 | 2000 |
| 93 | 250000 | 8 | 60 | 1000 |
| 94 | 8000 | 12 | 60 | 200 |
| 95 | 400000 | 12 | 70 | 2000 |
| 96 | 200000 | 12 | 70 | 1000 |
| 97 | 5000 | 24 | 80 | 200 |
| 98 | 500000 | 24 | 80 | 2000 |
| 99 | 250000 | 24 | 80 | 1000 |
| 100 | 4000 | 8 | 50 | 200 |
| 101 | 400000 | 8 | 50 | 2000 |
| 102 | 250000 | 8 | 60 | 1000 |
| 103 | 8000 | 12 | 60 | 200 |
| 104 | 400000 | 12 | 70 | 2000 |
| 105 | 200000 | 12 | 70 | 1000 |
| 106 | 5000 | 24 | 80 | 200 |

(continued)

| Composition Serial No. | Amount of water (ml) | Standing time (h) | Content of ethanol (%) | Amount of ethanol (ml) |
|---|---|---|---|---|
| 107 | 500000 | 24 | 80 | 2000 |
| 108 | 250000 | 24 | 80 | 1000 |
| 109 | 4000 | 8 | 50 | 200 |
| 110 | 400000 | 8 | 50 | 2000 |
| 111 | 250000 | 8 | 60 | 1000 |
| 112 | 8000 | 12 | 60 | 200 |
| 113 | 400000 | 12 | 70 | 2000 |
| 114 | 200000 | 12 | 70 | 1000 |
| 115 | 5000 | 24 | 80 | 200 |
| 116 | 500000 | 24 | 80 | 2000 |
| 117 | 250000 | 24 | 80 | 1000 |
| 118 | 4000 | 8 | 50 | 200 |
| 119 | 400000 | 8 | 50 | 2000 |
| 120 | 250000 | 8 | 60 | 1000 |
| 121 | 8000 | 12 | 60 | 200 |
| 122 | 400000 | 12 | 70 | 2000 |
| 123 | 200000 | 12 | 70 | 1000 |
| 124 | 5000 | 24 | 80 | 200 |
| 125 | 500000 | 24 | 80 | 2000 |
| 126 | 250000 | 24 | 80 | 1000 |
| 127 | 4000 | 8 | 50 | 200 |
| 128 | 400000 | 8 | 50 | 2000 |
| 129 | 250000 | 8 | 60 | 1000 |
| 130 | 8000 | 12 | 60 | 200 |
| 131 | 400000 | 12 | 70 | 2000 |
| 132 | 200000 | 12 | 70 | 1000 |
| 133 | 5000 | 24 | 80 | 200 |
| 134 | 500000 | 24 | 80 | 2000 |
| 135 | 250000 | 24 | 80 | 1000 |
| 136 | 4000 | 8 | 50 | 200 |
| 137 | 400000 | 8 | 50 | 2000 |
| 138 | 250000 | 8 | 60 | 1000 |
| 139 | 8000 | 12 | 60 | 200 |
| 140 | 400000 | 12 | 70 | 2000 |
| 141 | 200000 | 12 | 70 | 1000 |
| 142 | 5000 | 24 | 80 | 200 |
| 143 | 500000 | 24 | 80 | 2000 |
| 144 | 250000 | 24 | 80 | 1000 |

(continued)

| Composition Serial No. | Amount of water (ml) | Standing time (h) | Content of ethanol (%) | Amount of ethanol (ml) |
|---|---|---|---|---|
| 145 | 4000 | 8 | 50 | 200 |
| 146 | 400000 | 8 | 50 | 2000 |
| 147 | 250000 | 8 | 60 | 1000 |
| 148 | 8000 | 12 | 60 | 200 |
| 149 | 400000 | 12 | 70 | 2000 |
| 150 | 200000 | 12 | 70 | 1000 |
| 151 | 5000 | 24 | 80 | 200 |
| 152 | 500000 | 24 | 80 | 2000 |
| 153 | 250000 | 24 | 80 | 1000 |
| 154 | 4000 | 8 | 50 | 200 |
| 155 | 400000 | 8 | 50 | 2000 |
| 156 | 250000 | 8 | 60 | 1000 |
| 157 | 8000 | 12 | 60 | 200 |
| 158 | 400000 | 12 | 70 | 2000 |
| 159 | 200000 | 12 | 70 | 1000 |
| 160 | 5000 | 24 | 80 | 200 |
| 161 | 500000 | 24 | 80 | 2000 |
| 162 | 250000 | 24 | 80 | 1000 |
| 163 | 4000 | 8 | 50 | 200 |
| 164 | 400000 | 8 | 50 | 2000 |
| 165 | 250000 | 8 | 60 | 1000 |
| 166 | 8000 | 12 | 60 | 200 |
| 167 | 400000 | 12 | 70 | 2000 |
| 168 | 200000 | 12 | 70 | 1000 |
| 169 | 5000 | 24 | 80 | 200 |
| 170 | 500000 | 24 | 80 | 2000 |
| 171 | 250000 | 24 | 80 | 1000 |
| 172 | 4000 | 8 | 50 | 200 |
| 173 | 400000 | 8 | 50 | 2000 |
| 174 | 250000 | 8 | 60 | 1000 |
| 175 | 8000 | 12 | 60 | 200 |
| 176 | 400000 | 12 | 70 | 2000 |
| 177 | 200000 | 12 | 70 | 1000 |
| 178 | 5000 | 24 | 80 | 200 |
| 179 | 500000 | 24 | 80 | 2000 |
| 180 | 250000 | 24 | 80 | 1000 |

**Table 3 Composition Contents of Exemplary Embodiment 1-180**

| Composition Serial No. | Content of Rg3 and Rg5 in the composition | Proportion of each component in the composition (%) | | |
|---|---|---|---|---|
| | | s-Rg3 | R-Rg3 | Rg5 |
| 1 | 98.1% | 1 | 98 | 1 |
| 2 | 2.2% | 1 | 1 | 98 |
| 3 | 11.6% | 98 | 1 | 1 |
| 4 | 98.0% | 98 | 1 | 1 |
| 5 | 2.4% | 1 | 98 | 1 |
| 6 | 10.7% | 1 | 98 | 1 |
| 7 | 98.3% | 1 | 1 | 98 |
| 8 | 2.3% | 98 | 1 | 1 |
| 9 | 10.1% | 1 | 1 | 98 |
| 10 | 98.2% | 1 | 98 | 1 |
| 11 | 3.1% | 1 | 1 | 98 |
| 12 | 11.8% | 98 | 1 | 1 |
| 13 | 97.3% | 97 | 2 | 1 |
| 14 | 3.3% | 1 | 98 | 1 |
| 15 | 10.9% | 1 | 98 | 1 |
| 16 | 98.1% | 1 | 1 | 98 |
| 17 | 2.7% | 98 | 1 | 1 |
| 18 | 10.0% | 1 | 1 | 98 |
| 19 | 98.1% | 2 | 96 | 2 |
| 20 | 2.7% | 1 | 1 | 98 |
| 21 | 11.4% | 98 | 1 | 1 |
| 22 | 98.6% | 98 | 1 | 1 |
| 23 | 2.6% | 1 | 98 | 1 |
| 24 | 10.4% | 2 | 95 | 3 |
| 25 | 98.4% | 1 | 1 | 98 |
| 26 | 3.2% | 98 | 1 | 1 |
| 27 | 10.8% | 1 | 1 | 98 |
| 28 | 97.9% | 1 | 98 | 1 |
| 29 | 2.6% | 1 | 1 | 98 |
| 30 | 11.8% | 98 | 1 | 1 |
| 31 | 97.8% | 98 | 1 | 1 |
| 32 | 2.4% | 3 | 96 | 1 |
| 33 | 10.4% | 1 | 98 | 1 |
| 34 | 98.9% | 1 | 1 | 98 |
| 35 | 2.5% | 98 | 1 | 1 |
| 36 | 10.7% | 1 | 1 | 98 |

(continued)

| Composition Serial No. | Content of Rg3 and Rg5 in the composition | Proportion of each component in the composition (%) | | |
|---|---|---|---|---|
| | | s-Rg3 | R-Rg3 | Rg5 |
| 37 | 98.8% | 1 | 98 | 1 |
| 38 | 2.5% | 1 | 1 | 98 |
| 39 | 11.6% | 98 | 1 | 1 |
| 40 | 98.3% | 98 | 1 | 1 |
| 41 | 2.6% | 2 | 97 | 1 |
| 42 | 10.8% | 1 | 98 | 1 |
| 43 | 98.5% | 1 | 1 | 98 |
| 44 | 2.4% | 98 | 1 | 1 |
| 45 | 10.1% | 1 | 1 | 98 |
| 46 | 98.4% | 3 | 96 | 1 |
| 47 | 2.2% | 1 | 1 | 98 |
| 48 | 11.4% | 98 | 1 | 1 |
| 49 | 98.1% | 97 | 1 | 2 |
| 50 | 2.5% | 1 | 98 | 1 |
| 51 | 10.6% | 1 | 98 | 1 |
| 52 | 98.6% | 1 | 1 | 98 |
| 53 | 2.8% | 98 | 1 | 1 |
| 54 | 10.6% | 1 | 1 | 98 |
| 55 | 98.4% | 1 | 98 | 1 |
| 56 | 2.8% | 1 | 1 | 98 |
| 57 | 11.2% | 96 | 1 | 3 |
| 58 | 98.3% | 97 | 1 | 2 |
| 59 | 2.6% | 1 | 98 | 1 |
| 60 | 10.7% | 1 | 98 | 1 |
| 61 | 98.7% | 1 | 1 | 98 |
| 62 | 2.4% | 98 | 1 | 1 |
| 63 | 10.2% | 1 | 1 | 98 |
| 64 | 98.6% | 1 | 97 | 2 |
| 65 | 2.4% | 1 | 1 | 98 |
| 66 | 11.4% | 98 | 1 | 1 |
| 67 | 98.5% | 98 | 1 | 1 |
| 68 | 2.7% | 1 | 95 | 4 |
| 69 | 10.7% | 1 | 98 | 1 |
| 70 | 98.5% | 1 | 1 | 98 |
| 71 | 2.8% | 98 | 1 | 1 |
| 72 | 10.8% | 1 | 1 | 98 |

(continued)

| Composition Serial No. | Content of Rg3 and Rg5 in the composition | Proportion of each component in the composition (%) | | |
|---|---|---|---|---|
| | | s-Rg3 | R-Rg3 | Rg5 |
| 73 | 98.7% | 2 | 93 | 5 |
| 74 | 2.5% | 1 | 1 | 98 |
| 75 | 11.4% | 98 | 1 | 1 |
| 76 | 98.6% | 98 | 1 | 1 |
| 77 | 2.1% | 1 | 97 | 2 |
| 78 | 10.8% | 1 | 98 | 1 |
| 79 | 98.4% | 1 | 1 | 98 |
| 80 | 2.6% | 98 | 1 | 1 |
| 81 | 10.2% | 1 | 1 | 98 |
| 82 | 98.3% | 0 | 99 | 1 |
| 83 | 2.6% | 1 | 1 | 98 |
| 84 | 11.5% | 98 | 1 | 1 |
| 85 | 98.8% | 98 | 1 | 1 |
| 86 | 2.4% | 1 | 98 | 1 |
| 87 | 10.8% | 5 | 94 | 1 |
| 88 | 98.5% | 1 | 1 | 98 |
| 89 | 2.5% | 98 | 1 | 1 |
| 90 | 11.2% | 1 | 1 | 98 |
| 91 | 98.1% | 1 | 98 | 1 |
| 92 | 2.6% | 1 | 1 | 98 |
| 93 | 11.0% | 98 | 1 | 1 |
| 94 | 98.4% | 98 | 1 | 1 |
| 95 | 2.3% | 1 | 97 | 2 |
| 96 | 10.4% | 1 | 98 | 1 |
| 97 | 98.5% | 1 | 1 | 98 |
| 98 | 2.3% | 98 | 1 | 1 |
| 99 | 10.3% | 1 | 1 | 98 |
| 100 | 98.5% | 1 | 96 | 3 |
| 101 | 2.1% | 1 | 1 | 98 |
| 102 | 11.3% | 98 | 1 | 1 |
| 103 | 97.6% | 98 | 1 | 1 |
| 104 | 2.4% | 1 | 98 | 1 |
| 105 | 10.5% | 1 | 98 | 1 |
| 106 | 97.8% | 1 | 1 | 98 |
| 107 | 2.8% | 97 | 1 | 2 |
| 108 | 10.3% | 1 | 1 | 98 |

(continued)

| Composition Serial No. | Content of Rg3 and Rg5 in the composition | Proportion of each component in the composition (%) | | |
|---|---|---|---|---|
| | | s-Rg3 | R-Rg3 | Rg5 |
| 109 | 98.4% | 1 | 98 | 1 |
| 110 | 3.8% | 1 | 2 | 97 |
| 111 | 11.5% | 98 | 1 | 1 |
| 112 | 98.5% | 98 | 1 | 1 |
| 113 | 2.5% | 1 | 98 | 1 |
| 114 | 10.4% | 1 | 98 | 1 |
| 115 | 97.6% | 1 | 1 | 98 |
| 116 | 2.8% | 98 | 1 | 1 |
| 117 | 10.5% | 1 | 1 | 98 |
| 118 | 97.5% | 1 | 98 | 1 |
| 119 | 2.4% | 2 | 2 | 96 |
| 120 | 11.3% | 98 | 1 | 1 |
| 121 | 98.4% | 98 | 1 | 1 |
| 122 | 2.5% | 1 | 98 | 1 |
| 123 | 10.8% | 1 | 98 | 1 |
| 124 | 98.5% | 1 | 1 | 98 |
| 125 | 2.6% | 98 | 1 | 1 |
| 126 | 10.5% | 1 | 1 | 98 |
| 127 | 97.9% | 1 | 96 | 3 |
| 128 | 2.5% | 1 | 1 | 98 |
| 129 | 11.4% | 98 | 1 | 1 |
| 130 | 98.8% | 98 | 1 | 1 |
| 131 | 2.8% | 1 | 98 | 1 |
| 132 | 10.5% | 1 | 98 | 1 |
| 133 | 98.4% | 1 | 1 | 98 |
| 134 | 2.5% | 98 | 1 | 1 |
| 135 | 10.5% | 1 | 1 | 98 |
| 136 | 98.5% | 1 | 97 | 2 |
| 137 | 2.3% | 1 | 1 | 98 |
| 138 | 11.7% | 98 | 1 | 1 |
| 139 | 97.8% | 98 | 1 | 1 |
| 140 | 2.8% | 1 | 95 | 4 |
| 141 | 10.5% | 1 | 98 | 1 |
| 142 | 98.3% | 1 | 1 | 98 |
| 143 | 2.8% | 98 | 1 | 1 |
| 144 | 10.4% | 1 | 1 | 98 |

(continued)

| Composition Serial No. | Content of Rg3 and Rg5 in the composition | Proportion of each component in the composition (%) | | |
|---|---|---|---|---|
| | | s-Rg3 | R-Rg3 | Rg5 |
| 145 | 98.0% | 1 | 98 | 1 |
| 146 | 2.6% | 1 | 1 | 98 |
| 147 | 11.0% | 98 | 1 | 1 |
| 148 | 98.1% | 98 | 1 | 1 |
| 149 | 2.5% | 1 | 98 | 1 |
| 150 | 10.8% | 3 | 92 | 5 |
| 151 | 98.7% | 1 | 1 | 98 |
| 152 | 2.2% | 98 | 1 | 1 |
| 153 | 10.4% | 1 | 1 | 98 |
| 154 | 98.8% | 1 | 98 | 1 |
| 155 | 2.6% | 1 | 1 | 98 |
| 156 | 11.7% | 98 | 1 | 1 |
| 157 | 98.2% | 98 | 1 | 1 |
| 158 | 2.4% | 3 | 93 | 4 |
| 159 | 11.6% | 1 | 98 | 1 |
| 160 | 98.8% | 1 | 1 | 98 |
| 161 | 2.7% | 96 | 3 | 1 |
| 162 | 10.1% | 1 | 1 | 98 |
| 163 | 98.3% | 1 | 98 | 1 |
| 164 | 2.5% | 1 | 1 | 98 |
| 165 | 11.7% | 98 | 1 | 1 |
| 166 | 98.8% | 98 | 1 | 1 |
| 167 | 2.1% | 1 | 92 | 7 |
| 168 | 10.2% | 1 | 98 | 1 |
| 169 | 98.5% | 1 | 1 | 98 |
| 170 | 2.3% | 98 | 1 | 1 |
| 171 | 10.6% | 1 | 1 | 98 |
| 172 | 97.1% | 1 | 98 | 1 |
| 173 | 2.4% | 1 | 1 | 98 |
| 174 | 11.8% | 98 | 1 | 1 |
| 175 | 98.8% | 98 | 1 | 1 |
| 176 | 2.7% | 4 | 96 | 0 |
| 177 | 10.1% | 2 | 97 | 1 |
| 178 | 98.9% | 1 | 1 | 98 |
| 179 | 2.1% | 93 | 1 | 6 |
| 180 | 10.5% | 1 | 1 | 98 |

**Exemplary Embodiments 181-198: Preparation of the Composition of Ginsenoside Rg3 and Ginsenoside**

**Rg5 and the Composition of Cyclodextrins**

Take the composition of ginsenoside Rg3 and ginsenoside Rg5 prepared according to the process of Exemplary Embodiments 1-180 and prepare the composition at a weight ratio described in Table 4 and according to the following procedure: 1) directly add it to a cyclodextrin solution or a solution of cyclodextrin derivative; 2) directly add it to a cyclodextrin solution or a solution of cyclodextrin derivative and stirring thoroughly for 1-24 h; 3) directly add it to a cyclodextrin solution or a solution of cyclodextrin derivative and heating for 10-120 min; 4) directly add it to a cyclodextrin solution or a solution of cyclodextrin derivative and sonicate for 10-120 min; 5) directly add it to the cyclodextrin powder or the powder of cyclodextrin derivative and grind for 10-120 min; or 6) add the composition of ginsenoside Rg3 and ginsenoside Rg5 to the cyclodextrin powder or the powder of cyclodextrin derivative, mix well and sieve.

**Table 4 Raw Material Ratio and Preparation Method of Exemplary Embodiments 181-198**

| Exemplary Embodiment No. | Weight of composition (g, content) (Ginsenoside Rg3: ginsenoside Rg5) | □-Amount of cyclodextrin in gram | Preparation method |
|---|---|---|---|
| Exemplary embodiment 181 | 100 (98.1%)(2:98) | 100 | 1) Add directly |
| Exemplary embodiment 182 | 100 (98.1%)(2:98) | 10000 | 2) Stir for 1 h |
| Exemplary embodiment 183 | 100 (98.1%)(2:98) | 500 | 3) Heat for 10 min |
| Exemplary embodiment 184 | 100 (98.1%)(2:98) | 1000 | 4) Sonicate for 10 min |
| Exemplary embodiment 185 | 100 (98.1%)(2:98) | 2000 | 5) Grind for 10 min |
| Exemplary embodiment 186 | 100 (98.1%)(2:98) | 3000 | 6) Mix well and sieve for 10 min |
| Exemplary embodiment 187 | 100 (98.1%)(2:98) | 4000 | 1) Add directly |
| Exemplary embodiment 188 | 100 (98.1%)(2:98) | 3500 | 2) Stir for 12 h |
| Exemplary embodiment 189 | 100 (98.1%)(2:98) | 4500 | 3) Heat for 120 min |
| Exemplary embodiment 190 | 100 (98.1%)(2:98) | 1500 | 4) Sonicate for 120 min |
| Exemplary embodiment 191 | 100 (10.7%)(1:99) | 100 | 1) Add directly |
| Exemplary embodiment 192 | 100 (10.7%)(1:99) | 10000 | 2) Stir for 1 h |
| Exemplary embodiment 193 | 100 (10.7%)(1:99) | 500 | 3) Heat for 10 min |
| Exemplary embodiment 194 | 100 (10.7%)(1:99) | 1000 | 4) Sonicate for 10 min |
| Exemplary embodiment 195 | 100 (2.3%)(99:1) | 100 | 1) Add directly |
| Exemplary embodiment 196 | 100 (2.3%)(99:1) | 10000 | 2) Stir for 1 h |
| Exemplary embodiment 197 | 100 (2.3%)(99:1) | 500 | 3) Heat for 10 min |

(continued)

| Exemplary Embodiment No. | Weight of composition (g, content) (Ginsenoside Rg3: ginsenoside Rg5) | □-Amount of cyclodextrin in gram | Preparation method |
|---|---|---|---|
| Exemplary embodiment 198 | 100 (2.3%)(99:1) | 1000 | 4) Sonicate for 10 min |

The excipients in Exemplary Embodiment **181-198** are exemplified with selected-cyclodextrins, and other cyclodextrins and cyclodextrin derivatives are suitable for use in the present invention. Examples include 1) □-hydroxypropyl cyclodextrin, 2) hydroxyethyl-□-cyclodextrin, 3) 2,6-dimethyl-□-cyclodextrin, 4) 2,3,6-trimethyl-□-cyclodextrin, 5) 2,6-diethyl-□-cyclodextrin, 6) 2,3,6-triethyl-□-cyclodextrin, 7) maltosyl-□-cyclodextrin, 8) sulfobutylether β-cyclodextrin, 9) p-toluenesulfonyl chloride (p-TsCl) substituted β-cyclodextrin, 10) 6-position substituted β-CD p-toluenesulfonate (P-cyclodextrin-6-OTs), 11) 2-oxohydroxypropyl-β-cyclodextrin, 12) 2-position monosubstituted p-toluenesulfonate (2-β-cyclodextrin-2-OTs), 13) β-cyclodextrin p-toluenesulfonate (tosyl-β-CD) and 14) PCL-(Tos) 7-β-CD, the star-shaped macromolecule of β-cyclodextrin.

**Exemplary Embodiment 199: Preparation of Tablets of Composition of Ginsenoside Rg3 and Ginsenoside Rg5**

Prepare the tablets of composition of ginsenoside Rg3 and ginsenoside Rg5 according to the following ratios:

| | |
|---|---|
| A composition of ginsenoside Rg3 and ginsenoside Rg5 (both in a weight ratio of 2:98) | 500g |
| Starch | 480g |
| Talc | 1%(10g) |
| Magnesium stearate | 1%(10g) |

Take appropriate amounts of the composition of ginsenoside Rg3 and ginsenoside **Rg5** prepared by the process route in Exemplary Embodiments 1-180, add it with starch by the above ratios, mix well and prepare the mixture into granules, then add talc and magnesium stearate, mix well, and then compress the mixture into 10,000 tablets.

**Exemplary Embodiment 200: Preparation of Granules of Composition of Ginsenoside Rg3 and Ginsenoside Rg5**

Prepare the granules of the composition of ginsenoside Rg3 and ginsenoside Rg5 according to the following ratios:

| | |
|---|---|
| A composition of ginsenoside Rg3 and ginsenoside Rg5 (both in a weight ratio of 98:2) | 100g |
| Microcrystalline cellulose | 10000g |

Take an appropriate amount of the composition of ginsenoside Rg3 and ginsenoside Rg5 prepared by the process route in Exemplary Embodiments 1-180, add it with microcrystalline cellulose by the above ratios, mix well and prepare the mixture into granules and dispense into 10,000 sachets.

**Exemplary Embodiment 201: Preparation of Capsules of Composition of Ginsenoside Rg3 and Ginsenoside Rg5**

Prepare the capsules of the composition of ginsenoside Rg3 and ginsenoside Rg5 according to the following ratios:

| | |
|---|---|
| A composition of ginsenoside Rg3 and ginsenoside Rg5 (both in a weight ratio of 2:98) | 250g |
| Starch | 2500g |

Take an appropriate amount of the composition of ginsenoside Rg3 and ginsenoside Rg5 prepared by the process route in Exemplary Embodiments 1-180, add it with starch by the above ratios, mix well and dispense into 10,000 capsules.

**Exemplary Embodiments 202-205: Preparation of Capsules of Composition of Ginsenoside Rg3 and**

**Ginsenoside Rg5**

Take an appropriate amount of the composition of ginsenoside Rg3 and ginsenoside Rg5, add it with starch according to the weight ratios in Table 5, mix well and then dispense it into 10,000 capsules.

**Table 5 Raw Material Ratio of Exemplary Embodiments 202-205**

| Exemplary Embodiment No. | Weight of composition (g) (Ginsenoside Rg3: ginsenoside Rg5) | Excipient (Starch, g) | Weight ratio of raw material to excipient |
|---|---|---|---|
| Exemplary embodiment 202 | 500 (2:98) | 500 | 1:1 |
| Exemplary embodiment 203 | 50 (10:90) | 5000 | 1:100 |
| Exemplary embodiment 204 | 250 (25:75) | 2500 | 1:10 |
| Exemplary embodiment 205 | 250 (50:50) | 5000 | 1:20 |

The raw materials described in the table can be the composition of ginsenoside Rg3 and ginsenoside Rg5 in the Exemplary Embodiments 1-180

**Exemplary Embodiment 206-209: Preparation of Granules of Composition of Ginsenoside Rg3 and**

**Ginsenoside Rg5**

Take an appropriate amount of the composition of ginsenoside Rg3 and ginsenoside Rg5, add it with microcrystalline cellulose according to the weight ratios described in Table 6, mix well and prepare it into granules, then dispense it into 10,000 sachets.

**Table 6 Raw Material Ratios of Exemplary Embodiments 206-209**

| Exemplary Embodiment No. | Weight of composition (g) (Ginsenoside Rg3: ginsenoside Rg5) | Excipient (Microcrystalline cellulose, g) | Weight ratio of raw material to excipient |
|---|---|---|---|
| Exemplary embodiment 206 | 1000 (2:98) | 1000 | 1: 1 |
| Exemplary embodiment 207 | 250 (10:90) | 25000 | 1: 100 |
| Exemplary embodiment 208 | 2500 (25:75) | 25000 | 1: 10 |
| Exemplary embodiment 209 | 2500 (50:50) | 50000 | 1: 20 |

The raw materials described in the table can be the composition of ginsenoside Rg3 and ginsenoside Rg5 in the Exemplary Embodiments 1-180

**Exemplary Embodiment 210: Preparation of Tablets of Composition of Ginsenoside Rg3 and**

**Ginsenoside Rg5**

Prepare the tablets of composition of ginsenoside Rg3 and ginsenoside Rg5 according to the following ratios:

| | |
|---|---|
| A composition of ginsenoside Rg3 and ginsenoside Rg5 (both in a weight ratio of 98:2) | 500g |
| Starch | 380g |
| Licorice extract | 100g |
| Talc | 1%(10g) |
| Magnesium stearate | 1%(10g) |

Take an appropriate amount of the composition of ginsenoside Rg3 and ginsenoside Rg5, add it with starch by the above ratios, mix well and prepare the mixture into granules, then add talc and magnesium stearate, mix well, and then compress the mixture into 10,000 tablets. Wherein, the composition of ginsenoside Rg3 and ginsenoside Rg5 in this exemplary embodiment can be substituted by the composition of ginsenoside Rg3 and ginsenoside Rg5 in Exemplary Embodiment 1-180.

**Exemplary Embodiment 211: Preparation of Granules of Composition of Ginsenoside Rg3 and Ginsenoside Rg5**

Prepare the granules of the composition of ginsenoside Rg3 and ginsenoside Rg5 according to the following ratios:

| | |
|---|---|
| A composition of ginsenoside Rg3 and ginsenoside Rg5 (both in a weight ratio of 2:98) | 250g |
| Licorice extract | 250g |
| Microcrystalline cellulose | 24500g |

Take an appropriate amount of the composition of ginsenoside Rg3 and ginsenoside Rg5, add it with the above said extract powder by the above ratios, mix well and add it with microcrystalline cellulose and mix well; and then prepare the mixture into granules and dispense into 10,000 sachets. Wherein, the composition of ginsenoside Rg3 and ginsenoside Rg5 in this exemplary embodiment can be substituted by the composition of ginsenoside Rg3 and ginsenoside Rg5 in Exemplary Embodiment 1-180.

**Exemplary Embodiment 212: Preparation of Capsules of Composition of Ginsenoside Rg3 and Ginsenoside Rg5**

Prepare the capsules of the composition of ginsenoside Rg3 and ginsenoside Rg5 according to the following ratios:

| | |
|---|---|
| A composition of ginsenoside Rg3 and ginsenoside Rg5 (both in a weight ratio of 2:98) | 250g |
| Licorice extract | 250g |
| Extract of fritillariae cirrhosae bulbus | 250g |
| Extract of folium Ilicis latifoliae | 250g |
| Starch | 1000g |

Take an appropriate amount of the composition of ginsenoside Rg3 and ginsenoside Rg5, add it with the above said extract powder, mix well and add it with starch and mix well; and then dispense into 10,000 capsules.

Wherein, the composition of ginsenoside Rg3 and ginsenoside Rg5 in this exemplary embodiment can be substituted by the composition of ginsenoside Rg3 and ginsenoside Rg5 in Exemplary Embodiment 1-180.

**Exemplary Embodiment 213: Preparation of Tablets of Composition of Ginsenoside Rg3 and Ginsenoside Rg5**

Prepare the tablets of composition of ginsenoside Rg3 and ginsenoside Rg5 according to the following ratios:

| | |
|---|---|
| Composition of ginsenoside Rg3 and ginsenoside Rg5 (both in a weight ratio of 2:98) | 500g |
| Starch | 480g |

(continued)

| | |
|---|---|
| Extract of anemarrhenae rhizoma | 500g |
| Talc | 1%(10g) |
| Magnesium stearate | 1%(10g) |

Take an appropriate amount of the composition of ginsenoside Rg3 and ginsenoside Rg5, add it with the above said extract powder by the above ratios, mix well and add starch and mix well and prepare the mixture into granules, then add talc and magnesium stearate, mix well, and then compress the mixture into 10,000 tablets.

Wherein, the composition of ginsenoside Rg3 and ginsenoside Rg5 in this exemplary embodiment can be substituted by the composition of ginsenoside Rg3 and ginsenoside Rg5 prepared in Exemplary Embodiment 1-180.

**Exemplary Embodiment 214: Preparation of Granules of Composition of Ginsenoside Rg3 and Ginsenoside Rg5**

Prepare the granules of the composition of ginsenoside Rg3 and ginsenoside Rg5 according to the following ratios:

| | |
|---|---|
| A composition of ginsenoside Rg3 and ginsenoside Rg5 (both in a weight ratio of 10:90) | 1000g |
| Extract of scrophulariae radix | 500g |
| Extract lophatherum gracile | 500g |
| Microcrystalline cellulose | 10000g |

Take an appropriate amount of the composition of ginsenoside Rg3 and ginsenoside Rg5, add it with the above said extract powder by the above ratios, mix well and add it with microcrystalline cellulose and mix well; and then prepare the mixture into granules and dispense into 10,000 sachets. Wherein, the composition of ginsenoside Rg3 and ginsenoside Rg5 in this exemplary embodiment can be substituted by the composition of ginsenoside Rg3 and ginsenoside Rg5 prepared in Exemplary Embodiment 1-180.

**Exemplary Embodiment 215: Preparation of Solid Beverage of Composition of Ginsenoside Rg3 and Ginsenoside Rg5**

Prepare the solid beverage of the composition of ginsenoside Rg3 and ginsenoside Rg5 according to the following ratios:

| | |
|---|---|
| A composition of ginsenoside Rg3 and ginsenoside Rg5 (both in a weight ratio of 10:90) | 1000g |
| Powdered sugar | 5000g |

Take an appropriate amount of the composition of ginsenoside Rg3 and ginsenoside Rg5, add it with the above said extract powder, mix well and then prepare the mixture into granules and dispense into 10,000 sachets.

Wherein, the composition of ginsenoside Rg3 and ginsenoside Rg5 in this exemplary embodiment can be substituted by the composition of ginsenoside Rg3 and ginsenoside Rg5 prepared in Exemplary Embodiment 1-180.

**Exemplary Embodiment 216: Preparation of Compressed Confectionery Tablets of Composition of Ginsenoside Rg3 and Ginsenoside Rg5**

Prepare the solid beverage of the composition of ginsenoside Rg3 and ginsenoside Rg5 according to the following ratios:

| | |
|---|---|
| A composition of ginsenoside Rg3 and ginsenoside Rg5 (both in a weight ratio of 10:90) | 1000g |
| Powdered sugar | 5000g |

Take an appropriate amount of the composition of ginsenoside Rg3 and ginsenoside Rg5, add it with the above said extract powder, mix well and prepare it into granules and compress into tablets, then prepare 10,000 tablets. Wherein, the composition of ginsenoside Rg3 and ginsenoside Rg5 in this exemplary embodiment can be substituted by the composition of ginsenoside Rg3 and ginsenoside Rg5 prepared in Exemplary Embodiment 1-180.

**Exemplary Test 1 Immunity Boosting Effect of Ginsenoside Rg3 and Ginsenoside Rg5 in Mice**

**1. Experimental Materials**

1.1 Drugs and Reagents

The composition of ginsenoside Rg3 and ginsenoside Rg5 is produced by Dalian Fusheng Natural Drugs
Development Co., Ltd. The contents of Rg3 and Rg5 were determined by HPLC, respectively. See Table 7 for details.

**Table 7 List of Compositions Used in Exemplary Test 1**

| Compositi on Serial No. | Content of Rg3 and Rg5 in the composition | Proportion of each component in the composition | | |
|---|---|---|---|---|
| | | s-Rg3 | R-Rg3 | Rg5 |
| A | 98.1% | 1 | 98 | 1 |
| B | 2.2% | 1 | 1 | 98 |
| C | 11.6% | 98 | 1 | 1 |
| D | 98.0% | 98 | 1 | 1 |
| E | 2.4% | 1 | 98 | 1 |
| F | 10.7% | 1 | 98 | 1 |
| G | 98.3% | 1 | 1 | 98 |
| H | 2.3% | 98 | 1 | 1 |
| I | 10.1% | 1 | 1 | 98 |

Positive control drug: Pidotimod Oral Solution (Suzhou Pharmaceutical Factory of Jiangsu Wuzhong Pharmaceutical Group Corporation, strength: 10 ml/400 mg, lot No.: 2014091211)

1.2 Experimental Animals

Kunming mice, aged 6-8 weeks, weighing 18-22 g, were purchased from Experimental Animal Center of Dalian Medical University, with the quality certificate No.: SCXK (13) 2013-0003.

**2. Experimental Method**

2.1 Grouping and Administration

A total of 290 healthy male mice, as mentioned above, were subjected to acclimation for 4 days before they were randomly divided into 29 groups according to body weight, including the negative control group, the positive control group and ginsenoside composition experimental group, and the animals in the latter group were further divided into high dose group, medium dose group and low dose group, respectively. The animals of the positive control group were given pidotimod (50 mg/kg), those in the low dose ginsenoside composition group were given low dose ginsenoside composition (36 mg/kg), those in the medium dose group were given medium dose ginsenoside composition (72 mg/kg), those in the high dose group were given high dose of ginsenoside composition (144 mg/kg) and those in the negative control group were given the same volume of water. Drugs and water were administered once daily for 30 consecutive days.

2.2 Experiments and Results

**2.2.1 ConA-induced Mouse Splenic Lymphocyte Transformation Test**

At 1 h after the last dose, the spleens of animals of each group were aseptically excised to prepare splenocyte suspension. After diluting the splenocyte suspension to a concentration of $3 \times 10^6$ cells/mL, the splenocyte suspension was divided into 2 aliquots. Each of the aliquot was transferred into two 24-well culture plates, respectively at a volume of 1 mL/well, and then 75 $\mu$L ConA solution (a) was added to a well, and the other well was used as control (b), which were cultured at 37°C for 72 h. Thiazolyl blue (MTT) was added 4 h before the end of the culture. After incubation, acidic isopropanol was added, and the absorbance (ABS) of each solution was measured at 570 nm after mixing well. The proliferativity was calculated using the formula (proliferativity = ABSa-ABSb). Each dose group of the test sample was compared with the negative control group. The experimental results are shown in Table 1.

**2.2.2 Determination of NK Cell Viability**

At 1 h after the last dose, the spleens of animals of each group were aseptically excised to prepare splenocyte suspension. After the red blood cells were lysed with sterile water for injection, the cell suspension was diluted with 1% glacial acetic acid. Then, after adjusting the concentration of splenocyte suspension to $2\times10^7$ cells/mL, the splenocyte suspension was added to a 96-well plate for culturing. For each animal, the wells were divided into reaction wells (splenocyte suspension and YAC-1 cell suspension 100 $\mu$L each; effector-to-target ratio was 50:1); natural release wells (YAC-1 cell suspension and culture medium 100 $\mu$L each) and maximum release wells (YAC-1 cell suspension and 1% NP40 100 $\mu$L each). A triplicate set of tubes was prepared for each of the above items. The 96-well plate was incubated at 37°C in a 5% $CO_2$ incubator for 4 h. then, after addition of LDH matrix solution and 1 moL/LHCl, the solutions in each parallel well were combined, and the absorbance (ABS) was measured at 490 nm. Then, the NK cell viability was calculated. NK cell viability (%) = (ABS reaction well -ABS $_{natural\ release\ well}$)/(ABS maximum release well -ABS natural release well). The experimental results are shown in Table 8.

**Table 8 Effect of Ginsenoside Composition on NK Cell Viability and ConA-induced Lymphocyte Proliferation Ability (x $\pm$ s)**

| Group | | Dose administered (mg/kg) | NK cell viability (%) | Proliferation ability ($\times10^{-2}$) |
|---|---|---|---|---|
| Negative control group | | -- | 34.14$\pm$5.68 | 9.04$\pm$3.81 |
| Positive control group | | 50 | 42.57$\pm$7.31 | 27.97$\pm$11.45 |
| Composition A | Low dose group | 36 | 64.14$\pm$5.40 | 44.27$\pm$5.52 |
| | Medium dose group | 72 | 70.04$\pm$8.32 | 50.14$\pm$9.59 |
| | High dose group | 144 | 76.53$\pm$7.33 | 58.69$\pm$11.23 |
| Composition B | Low dose group | 36 | 75.55$\pm$9.15 | 27.99$\pm$8.03 |
| | Medium dose group | 72 | 48.53$\pm$10.53 | 28.61$\pm$10.12 |
| | High dose group | 144 | 51.73$\pm$8.76 | 31.99$\pm$16.87 |
| Composition C | Low dose group | 36 | 53.56$\pm$6.84 | 33.94$\pm$10.60 |
| | Medium dose group | 72 | 55.82$\pm$6.29 | 35.44$\pm$11.74 |
| | High dose group | 144 | 57.54$\pm$11.97 | 37.64$\pm$13.61 |
| Composition D | Low dose group | 36 | 64.78$\pm$5.40 | 44.32$\pm$6.22 |
| | Medium dose group | 72 | 70.54$\pm$10.24 | 50.02$\pm$5.24 |
| | High dose group | 144 | 76.84$\pm$9.33 | 58.51$\pm$4.98 |
| Composition E | Low dose group | 36 | 45.78$\pm$9.15 | 27.95$\pm$7.99 |
| | Medium dose group | 72 | 47.45$\pm$10.53 | 27.33$\pm$9.28 |
| | High dose group | 144 | 50.94$\pm$8.47 | 30.52$\pm$7.53 |

(continued)

| Group | | Dose administered (mg/kg) | NK cell viability (%) | Proliferation ability ($\times 10^{-2}$) |
|---|---|---|---|---|
| Composition F | Low dose group | 36 | 53.78±6.84 | 33.64±8.57 |
| | Medium dose group | 72 | 55.49±11.29 | 35.46±10.68 |
| | High dose group | 144 | 57.65±7.97 | 37.48±9.24 |
| Composition G | Low dose group | 36 | 65.77±5.40 | 45.68±8.57 |
| | Medium dose group | 72 | 71.65±8.32 | 51.71±8.53 |
| | High dose group | 144 | 77.95±7.38 | 59.61±8.64 |
| Composition H | Low dose group | 36 | 43.88±9.15 | 27.35±9.63 |
| | Medium dose group | 72 | 47.45±9.01 | 28.96±8.59 |
| | High dose group | 144 | 50.46±5.37 | 30.57±9.85 |
| Composition I | Low dose group | 36 | 54.14±8.44 | 34.94±6.87 |
| | Medium dose group | 72 | 56.95±11.29 | 36.08±10.68 |
| | High dose group | 144 | 58.74±7.97 | 38.84±12.60 |

**3. Test Results**

Following the stimulation of T lymphocytes by ConA, the blast cells will show proliferative responses. The mitochondrial hydrolase in the viable cells, especially the proliferating cells, decomposes MTT into blue-purple crystals. If the optical density value is increased, it indicates that the cell proliferation ability is enhanced. As shown in Table 1, the optical density differences of the high-, medium- and low-dose groups of the ginsenoside composition were higher than those of the negative control group, indicating that this sample can promote the proliferation of splenocytes.

After the cells are killed by the NK cells, the LDH in the cytoplasm of viable cells will be released to the outside of the cells, and LDH can dehydrogenate lithium lactate, so that NAD is reduced to NADH, which in turn is reduced to iodonitro-tetrazolium chloride (INT) through hydrogen transmitter phenazine methosulfate (PMS). After receiving H+, the INT is reduced to a purple formazan compound and the optical density value is determined using a microplate reader. As shown in Table 1, the NK cell viability of the high-, medium- and low-dose groups of the ginsenoside composition was significantly higher than that of the negative control group, indicating that the sample can increase the viability of the NK cells.

The above experimental results showed that all the different doses of the composition of ginsenoside Rg3 and ginsenoside Rg5 could promote the proliferation of splenocytes, increase the viability of the NK cells, and could significantly improve the immune function.

**Exemplary Test 2 Anti-tumor Effect of Ginsenoside Composition on S180 Sarcoma in Mice**

**1. Experimental Materials**

**1.1 Experimental Animals and Transplanted Tumor Strains**

Kunming mice, provided by Guangdong Medical Laboratory Animal Center, with certificate No.: SCXK2013-005;

S180 sarcoma cells, purchased from Guangdong Medical Laboratory Animal Center.

**1.2 Drug Product**

The composition of ginsenoside Rg3 and ginsenoside Rg5 is produced by Dalian Fusheng Natural Drugs Development Co., Ltd. The contents of Rg3 and Rg5 were determined by HPLC, respectively.

**Table 9 List of Compositions Used in Exemplary Test 2**

| Composition Serial No. | Content of Rg3 and Rg5 in the composition | Proportion of each component in the composition | | |
|---|---|---|---|---|
| | | s-Rg3 | R-Rg3 | Rg5 |
| A | 98.1% | 1 | 98 | 1 |
| B | 2.2% | 1 | 1 | 98 |
| C | 11.6% | 98 | 1 | 1 |
| D | 98.0% | 98 | 1 | 1 |
| E | 2.4% | 1 | 98 | 1 |
| F | 10.7% | 1 | 98 | 1 |
| G | 98.3% | 1 | 1 | 98 |
| H | 2.3% | 98 | 1 | 1 |
| I | 10.1% | 1 | 1 | 98 |

The low, medium and high doses of the investigational product were 2 times, 4 times and 8 times the clinical equivalent dose for adults (calculated according to the body weight of 60 kg of adults), respectively. The suspension at the required concentration was prepared with double distilled water. The prepared drug solution was stored in a refrigerator at 4°C and protected from light until use.

The positive control drug is cyclophosphamide (lot No.: 20130105, Jiangsu Hengrui Medicine Co., Ltd.). For the clinical equivalent dose for the adults (calculated according to the body weight of 60 kg of adults), the required suspension was prepared with double distilled water and stored in a refrigerator at 4°C and protected from light until use.

**2. Experimental Method**

**2.1 Grouping and Administration**

Mice inoculated with sarcoma 180 (S180) ascites tumor cells which were well grown for 7 days were taken and the ascites fluid was aseptically collected, then diluted with sterile normal saline to adjust the cell number to $2 \times 10^6/0.2$ mL and stored in ice water until use.

A total of 300 healthy Kunming mice (half male and half female) aged 6-8 weeks and weighing 18-22 g were preliminarily housed for 1 week under the experimental conditions. After that, 10 of the animals were randomly selected as the blank control group, and the remaining 290 mice were subcutaneously inoculated with 0.2 mL of S 180 tumor cell suspension in the axilla of the left hind limb of each mouse. Twenty-four hours following the inoculation, the mice were weighed and randomly divided into 29 groups, i.e., the tumor-bearing control group, positive control group and ginsenoside composition experimental group. And the animals in the latter group were further divided into the high dose groups, medium dose groups and low dose groups, respectively. Starting from the grouping day, animals in the low dose group (36 mg/kg), medium dose group (72 mg/kg) and high dose group (144 mg/kg) of the ginsenosides composition and the positive control group were administered with cyclophosphamide 45 mg/kg via oral gavage. Animals in the normal control group and the model group were given equivalent amount of normal saline via oral gavage once daily.

After modeling and medication, the food intake, water intake, changes in coat color, activity, response to stimulation, presence of diarrhea, emaciation and death of mice in each group were observed and recorded at any time. Drugs were discontinued on the 21st day after oral gavage administration. On the next day, the mice were sacrificed by cervical dislocation and weighed. The subcutaneous tumor tissues of the left hind limb were completely stripped and weighed using an electronic balance after removing all the non-tumor tissues, such as blood stain and fat. The tumor inhibition rate was calculated according to the following formula:

Tumor inhibition rate = (average tumor weight of control group-average tumor weight of treatment group)/average tumor weight of control group $\times$ 100%

2.2 The data were expressed as mean $\pm$ standard deviation ($\pm$ s). The inter-group comparison of the measurement data was subjected to one-way analysis of variance using SPASS 13.0 statistical software. p<0.05 indicates statistically significant differences.

## 3. Experimental Results

### 3.1 Observation of General Conditions of Animals after Administration

After administration, the mice in all the groups were alive; the mice in the blank control group had normal food and water intake, flexible movement, quick reaction, shiny hair coat, and had no diarrhea or emaciation. In comparison, the general condition of the mice in each dose group of ginsenoside composition was inferior to those of the blank control group. Mice in the positive control group had poor food and water intake, slow movement, slow reaction, shedding and dull hair coat color, diarrhea and emaciation. The general conditions of the mice in the tumor-bearing control group were the worst, with little food intake and activity, slow reaction, haggard hair coat, and obvious emaciation.

3.2 The weight of transplanted tumors and tumor inhibition rates of the mice in each group are shown in Table 10.

**Table 10 Weight of Transplanted Tumors and Tumor Inhibition Rates of Mice in Each Group**

| Group | | Dose mg/kg/d | Number of animals (animal) Start/End | Weight of transplanted tumor (g) $\pm$ S | Tumor inhibition rate % |
|---|---|---|---|---|---|
| Blank control | | --- | 10/10 | --- | --- |
| Tumor-bearing control | | --- | 10/10 | 3.66±0.59 | --- |
| Positive control group | | 45 | 10/10 | 2.45±0.62 | 33.06 |
| Ginsenoside composition A | Low dose group | 36 | 10/10 | 1.76±084. | 51.91 |
| | Medium dose group | 72 | 10/10 | 1.57±0.62 | 57.1 |
| | High dose group | 144 | 10/10 | 1.21±1.54 | 66.94 |
| Ginsenoside composition B | Low dose group | 36 | 10/10 | 2.48±0.64 | 32.24 |
| | Medium dose group | 72 | 10/10 | 2.36±1.31 | 35.52 |
| | High dose group | 144 | 10/10 | 2.26±0.81 | 38.25 |
| Ginsenoside composition C | Low dose group | 36 | 10/10 | 2.15±0.89 | 41.25 |
| | Medium dose group | 72 | 10/10 | 2.06±0.98 | 43.72 |
| | High dose group | 144 | 10/10 | 1.99±1.04 | 45.63 |
| Ginsenoside composition D | Low dose group | 36 | 10/10 | 1.77±0.69 | 51.64 |
| | Medium dose group | 72 | 10/10 | 1.61±1.06 | 56.01 |
| | High dose group | 144 | 10/10 | 1.24±1.09 | 66.12 |

(continued)

| Group | | Dose mg/kg/d | Number of animals (animal) Start/End | Weight of transplanted tumor (g) $\pm$ S | Tumor inhibition rate % |
|---|---|---|---|---|---|
| Ginsenoside composition E | Low dose group | 36 | 10/10 | 2.52±0.57 | 31.15 |
| | Medium dose group | 72 | 10/10 | 2.39±0.68 | 34.70 |
| | High dose group | 144 | 10/10 | 2.29±1.69 | 37.43 |
| Ginsenoside composition F | Low dose group | 36 | 10/10 | 2.14±0.97 | 41.53 |
| | Medium dose group | 72 | 10/10 | 2.09±0.86 | 42.90 |
| | High dose group | 144 | 10/10 | 2.00±1.24 | 45.36 |
| Ginsenoside composition G | Low dose group | 36 | 10/10 | 1.72±0.76 | 53.00 |
| | Medium dose group | 72 | 10/10 | 1.55±0.81 | 57.65 |
| | High dose group | 144 | 10/10 | 1.24±0.67 | 66.12 |
| Ginsenoside Combination H | Low dose group | 36 | 10/10 | 2.50±0.65 | 31.69 |
| | Medium dose group | 72 | 10/10 | 2.39±1.21 | 34.70 |
| | High dose group | 144 | 10/10 | 2.29±0.59 | 37.43 |
| Ginsenoside composition I | Low dose group | 36 | 10/10 | 2.15±0.54 | 41.26 |
| | Medium dose group | 72 | 10/10 | 2.08±0.58 | 43.17 |
| | High dose group | 144 | 10/10 | 1.98±0.89 | 45.90 |

The experimental results showed that the weight of transplanted tumors in mice of the treatment group was significantly lower than those in the control group, indicating that the ginsenoside compositions A, B, C had an anti-tumor effect on transplanted S180 sarcoma in mice, and the tumor inhibition rates of ginsenoside compositions A, D, G were greater than 50%, which sufficiently indicated that the ginsenoside compositions had prominent tumor inhibiting effect.

**Exemplary Test 3: Ginsenoside Composition Improves Resistance to Anti-tumor Targeted Drugs**

**1. Materials and Methods**

Human cholangiocarcinoma cell line QBC939, purchased from Shanghai Honshun Biological Technology Co., Ltd.

For the ginsenoside composition produced by Dalian Fusheng Natural Drugs Development Co., Ltd., see Table 11 for details.

**Table 11 List of Compositions Used in Exemplary Test 3**

| Composition Serial No. | Content of Rg3 and Rg5 in the composition | Proportion of each component in the composition | | |
|---|---|---|---|---|
| | | s-Rg3 | R-Rg3 | Rg5 |
| A | 98.1% | 1 | 98 | 1 |
| B | 2.2% | 1 | 1 | 98 |
| C | 11.6% | 98 | 1 | 1 |
| D | 98.0% | 98 | 1 | 1 |
| E | 2.4% | 1 | 98 | 1 |
| F | 10.7% | 1 | 98 | 1 |
| G | 98.3% | 1 | 1 | 98 |
| H | 2.3% | 98 | 1 | 1 |
| I | 10.1% | 1 | 1 | 98 |

**2. Experimental Method**

**2.1 Construction of Drug-resistant Cancer Cell Lines**

The human cholangiocarcinoma cell line QBC939 was thawed and inoculated into T25 cell culture flasks filled with cell culture medium, and QBC939/ADM was constructed by continuously increasing the contact concentration of ADM.

**2.2 CCK-8 Assay in Detecting Cytotoxicity of Chemotherapeutic Agents**

The cells were divided into a blank zeroed control group, a normal control group and 9 experimental groups, each containing 100 $\mu$L of 80 $\mu$g/mL ginsenoside composition for cell culturing.

The control group (chemotherapeutic drug group): 100 $\mu$L of culture medium containing chemotherapeutic drugs was added to each well. The chemotherapeutic drugs included the targeted drugs afatinib (400 $\mu$g/mL), ceritinib (1000 $\mu$g/mL) and erlotinib (200 $\mu$g/mL). Six replicate wells were set up for each drug.

The experimental group (chemotherapeutic drugs + traditional Chinese medicine): 100 $\mu$L of culture medium containing chemotherapeutic drugs + ginsenoside composition was added to each well, namely afatinib (400 $\mu$g/mL) + ginsenoside composition (80 $\mu$g/mL), ceritinib (1000 $\mu$g/mL) + ginsenoside composition (80 $\mu$g/mL), and erlotinib (200 $\mu$g/mL) + ginsenoside composition (80 $\mu$g/mL). Six replicate wells were set up for each drug.

**3. Experimental Results**

The cytotoxic effects of the chemotherapeutic drugs were detected 24 h later. The results showed that the OD value (absorbance) of the cells in the experimental group was significantly lower than that in the control group, indicating that the viability of the cells in the experimental group was significantly lower than that in the control group, and the cytotoxic effect of the chemotherapeutic drugs on the cells in the experimental group was significantly more potent than that in the control group, indicating that the ginsenoside composition experimental group had the effect of reversing drug resistance. The results are shown in Table 12.

**Table 12 Absorbance Values in Drug Resistance Test**

| Group | Afatinib | Ceritinib | Erlotinib |
|---|---|---|---|
| Control group | 1.179±0.147 | 1.304±0.096 | 1.294±0.131 |
| Composition A | 0.703±0.132 | 0.611±0.101 | 0.608±0.122 |
| Composition B | 1.002±0.102 | 0.924±0.122 | 0.913±0.135 |
| Composition C | 0.950±0.141 | 0.840±0.139 | 0.837±0.095 |

(continued)

| Group | Afatinib | Ceritinib | Erlotinib |
|---|---|---|---|
| Composition D | 0.706±0.127 | 0.613±0.092 | 0.604±0.134 |
| Composition E | 1.016±0.135 | 0.914±0.137 | 0.902±0.127 |
| Composition F | 0.943±0.129 | 0.856±0.121 | 0.844±0.131 |
| Composition G | 0.691±0.136 | 0.573±0.126 | 0.562±0.134 |
| Composition H | 1.013±0.097 | 0.930±0.127 | 0.823±0.098 |
| Composition I | 0.941±0.087 | 0.852±0.093 | 0.841±0.096 |

Exemplary Test 4: Experiment of Ginsenoside Composition in Relieving Side Effects (Vomiting) of Radiotherapy and Chemotherapy

**1. Experimental Materials**

**1.1 Drugs and Reagents:**

The ginsenoside composition is white to brownish yellow powder manufactured by Dalian Fusheng Natural Drugs Development Co., Ltd. Its content was determined by high performance liquid chromatography (HPLC) with two kinds of detectors and UV detector and using the reference standard of National Institute for the Control of Pharmaceutical and Biological Products as the external standard. See Table 13 for details.

**Table 13 List of Compositions Used in Exemplary Test 4**

| Composition Serial No. | Content of Rg3 and Rg5 in the composition | Proportion of each component in the composition | | |
|---|---|---|---|---|
| | | s-Rg3 | R-Rg3 | Rg5 |
| A | 98.1% | 1 | 98 | 1 |
| B | 2.2% | 1 | 1 | 98 |
| C | 11.6% | 98 | 1 | 1 |
| D | 98.0% | 98 | 1 | 1 |
| E | 2.4% | 1 | 98 | 1 |
| F | 10.7% | 1 | 98 | 1 |
| G | 98.3% | 1 | 1 | 98 |
| H | 2.3% | 98 | 1 | 1 |
| I | 10.1% | 1 | 1 | 98 |

Positive control drug: metoclopramide Injection (Tianjin Renmin Pharmaceutical Factory, strength: 1 mL/10 mg, lot No.: 14060129);

Kaolin (Shanghai Fengxian Fengcheng Reagent Factory, lot No.: 140528);
Preparation of kaolin for rats: 1 g of acacia gum was weighed and added with 100 mL of distilled water, then it was placed on a magnetic stirrer and stirred until completely dissolved; then an appropriate amount of kaolin which has been sieved through a 80 mesh sieve was taken and slowly added into the above solution while stirring, allowing it to form into a dough-like shape. Then, it was squeezed into a 5 mL syringe until the syringe was completely filled; then the plunger rod was pushed to prepare small rods with very smooth edges with the shape resembling that of normal foods; then the rods were placed into a dryer filled with blue silica gel particles and allowed to dry for later use.

**1.2 Experimental Animals:**

Male Wistar rats, weighing 200-220 g, were purchased from the Experimental Animal Center of Dalian Medical University, with quality certificate No.: SCXK (13) 2013-0002.

**2. Experimental Method**

Rats are rodents with no vomiting reactions. The pica behavior of rats after vestibular stimulation is equivalent to the reaction of vomiting of other animals, which can be regarded as a typical symptom of motion sickness in rats.

2-1. Breeding of Rats for Acclimation

Before the initiation of the experiment, the rats were bred for acclimation, that is, the quantified routine feeds and kaolin feed were given to the rats simultaneously. Two kinds of feeds were taken out and weighed at regular time points every day to observe bite marks on the feed surface until the rats almost no longer gnawed the kaolin, and then the formal experiment was started.

2-2 Pica Test

Wistar rats were randomly divided into 29 groups (with 6 rats in each group), namely the model control group, the positive drug control group, the ginsenoside composition experimental group and the last group was further divided into three groups, i.e., the low-, medium- and high-dose groups.
The rats in the model control group were given saline at 10 mL/kg via oral gavage; those in the positive control group were intraperitoneally injected with metoclopramide at 2 mg/kg; and those in the low-, medium-and high-dose groups of ginsenoside composition were administered with ginsenoside compositions A-I at 15 mg/kg, 30 mg/kg and 60 mg/kg, respectively.
At 30 min post-dose, rotatory stimulation was applied using the rotatory stimulation device modified from a centrifuge to induce motion sickness, with the rotatory speed of 100 rotations/min and rotate for 1 h.
The rats of each group were observed for the kaolin ingestion pica behavior and the consumption of routine diet and kaolin was recorded within 24 h after the rotatory stimulation was stopped.

**3. Test Results**

All the data were expressed as mean $\pm$ standard deviation (X $\pm$ S), and the PPMS medical statistical software was used for the analysis of variance and the significance test by comparing the P values.
The statistical results of the consumption of routine diet and kaolin are shown in Table 14.
**Table 14 Effect of Ginsenoside Compositions on Rotation-induced Pica Behavior in Rats (X $\pm$ s, n=6)**

| Group | | Kaolin intake (g) | Food intake (g) |
|---|---|---|---|
| Blank control group | | 0.601±0.097 | 12.96±1.03 |
| Positive control group | | 0.404±0.131 | 23.97±1.16 |
| Ginsenoside composition A | Low dose group | 0.347±0.073 | 22.13±1.67 |
| | Medium dose group | 0.321±0.101 | 24.96±1.90 |
| | High dose group | 0.314±0.042 | 26.92±1.52 |
| Ginsenoside composition B | Low dose group | 0.473±0.064 | 14.29±1.36 |
| | Medium dose group | 0.439±0.093 | 15.37±1.91 |
| | High dose group | 0.413±0.065 | 16.48±0.93 |
| Ginsenoside composition C | Low dose group | 0.381±0.082 | 17. 13±2.71 |
| | Medium dose group | 0.369±0.094 | 18.52±1.94 |
| | High dose group | 0.362±0.113 | 20.67±0.99 |

(continued)

| Group | | Kaolin intake (g) | Food intake (g) |
|---|---|---|---|
| Ginsenoside composition D | Low dose group | 0.347±0.072 | 22.43±1.63 |
| | Medium dose group | 0.319±0.061 | 25.32±1.84 |
| | High dose group | 0.310±0.057 | 26.67±1.57 |
| Ginsenoside composition E | Low dose group | 0.464±0.119 | 14.03±1.73 |
| | Medium dose group | 0.428±0.070 | 15.88±0.91 |
| | High dose group | 0.415±0.054 | 16.93±1.22 |
| Ginsenoside composition F | Low dose group | 0.384±0.077 | 17.81±1.53 |
| | Medium dose group | 0.377±0.113 | 18.59±1.04 |
| | High dose group | 0.365±0.085 | 20.14±1.01 |
| Ginsenoside composition G | Low dose group | 0.346±0.090 | 22.38±1.73 |
| | Medium dose group | 0.325±0.041 | 25.52±1.86 |
| | High dose group | 0.318±0.086 | 26.27±1.42 |
| Ginsenoside composition H | Low dose group | 0.469±0.044 | 14.63±1.27 |
| | Medium dose group | 0.435±0.082 | 15.27±0.84 |
| | High dose group | 0.410±0.059 | 16.84±1.27 |
| Ginsenoside composition I | Low dose group | 0.383±0.105 | 17.35±1.65 |
| | Medium dose group | 0.379±0.064 | 19.46±1.21 |
| | High dose group | 0.360±0.097 | 20.74±0.98 |

The experimental results showed that:

The main side effect of radiotherapy and chemotherapy was vomiting, which was reflected in the pica behavior of kaolin ingestion of the rats. In each of the ginsenoside composition dose group, the pica behavior of kaolin ingestion of the rats was significantly inhibited, and the amount of kaolin intake was significantly decreased compared with the blank control group ($P < 0.05$, $P < 0.01$). In each dose group of the ginsenoside composition,
the treatment could significantly increase the food intake of the rats. The results of the above experiments showed that ginsenoside composition has a significant effect in alleviating emesis caused by radiotherapy and chemotherapy. Hence, the composition has good prospects for clinical application.

**Examplary Test 5: Anti-fatigue Effect of Ginsenoside Composition**

**1. Experimental Materials**

**1.1 Drugs and Reagents**

SOD Assay Kit, Jiancheng Biotechnology Co., Ltd.

The composition of ginsenoside Rg3 and ginsenoside Rg5, lot No.: 20120316, was produced by Dalian Fusheng Natural Drugs Development Co., Ltd. The contents of Rg3 and Rg5 were determined by HPLC, respectively, using the reference standard provided by the National Institute for the Control of Pharmaceutical and Biological Products.

**Table 17 List of Compositions Used in Examplary Test 6**

| Composition Serial No. | Content of Rg3 and Rg5 in the composition | Proportion of each component in the composition | | |
|---|---|---|---|---|
| | | s-Rg3 | R-Rg3 | Rg5 |
| A | 98.1% | 1 | 98 | 1 |

(continued)

| Composition Serial No. | Content of Rg3 and Rg5 in the composition | Proportion of each component in the composition | | |
|---|---|---|---|---|
| | | s-Rg3 | R-Rg3 | Rg5 |
| B | 2.2% | 1 | 1 | 98 |
| C | 11.6% | 98 | 1 | 1 |
| D | 98.0% | 98 | 1 | 1 |
| E | 2.4% | 1 | 98 | 1 |
| F | 10.7% | 1 | 98 | 1 |
| G | 98.3% | 1 | 1 | 98 |
| H | 2.3% | 98 | 1 | 1 |
| I | 10.1% | 1 | 1 | 98 |

### 1.2 Experimental Animals:

BALB/C nude mice, weighing 26-28 g, aged 6 weeks, were purchased from Beijing Vital River Laboratory Animal Technology Co., Ltd.

### 2. Experimental Method

### 2.1 Grouping and Administration:

A total of 280 selected male mice were firstly bred for 8 weeks, bred for acclimation for 1 week and then provided with exercise training for 1 week.

They were randomly divided into 28 groups according to body weight, including the control group and the ginsenoside composition experimental groups, and animals in the latter group were further divided into high dose group, medium dose group and low dose group, respectively.

Animals in the control group were given normal saline; those in the low dose ginsenoside composition group were given low dose of ginsenoside composition (3.2 mg/kg); those in the medium dose group were given medium dose of ginsenoside composition (6.4 mg/kg); and those in the high dose group were given high dose of ginsenoside composition (12.8 mg/kg). The drug was administered once daily for 30 consecutive days.

### 2.3 Experiments and Results

### Exhaustive Swimming Test

The dimension of the swimming pool is 185 cm × 73 cm × 58 cm, and it was 30 cm deep when the animals were swimming, and the water temperature was 27 ± 1°C. Nude mice were put into the swimming pool, and the time interval from going into the water to swim to the time of exhaustion was recorded. The judgment standard for exhaustion is that the mice demonstrate obvious movement disorder, with the head sinking into the water and failing to rise above the water surface for 8 s.

### Determination of SOD Activity

The activity of SOD in the blood is an important indicator of the body's anti-fatigue ability. It can be used to evaluate the fatigue index.

#### Table 18 Exhaustive Swimming Test and SOD Data Sheet

| Group | Dose (mg/kg) | Swimming duration (s) | SOD activity (u/mL) |
|---|---|---|---|
| Blank control group | - | 91.21±6.4 | 60.56±6.4 |

(continued)

| Group | Dose (mg/kg) | Swimming duration (s) | SOD activity (u/mL) |
|---|---|---|---|
| Composition A | 12.8 | 119.0±3.1 | 93.7±4.1 |
| | 6.4 | 113.4±2.1 | 90.3±5.9 |
| | 3.2 | 106.2±2.2 | 87.2±4.3 |
| Composition B | 12.8 | 95.1±5.3 | 75.7±6.2 |
| | 6.4 | 94.8±2.7 | 74.6±2.8 |
| | 3.2 | 93.0±3.4 | 73.4±5.4 |
| Composition C | 12.8 | 101.3±6.5 | 83.3±6.3 |
| | 6.4 | 98.2±3.3 | 81.5±4.7 |
| | 3.2 | 96.5±4.7 | 80.7±5.5 |
| Composition D | 12.8 | 119.2±3.6 | 93.8±3.4 |
| | 6.4 | 113.1±4.1 | 90.4±1.6 |
| | 3.2 | 105.8±5.9 | 87.7±5.6 |
| Composition E | 12.8 | 95.7±5.8 | 75.8±6.5 |
| | 6.4 | 94.4±3.2 | 74.7±3.5 |
| | 3.2 | 93.9±3.1 | 73.3±5.7 |
| Composition F | 12.8 | 101.6±5.0 | 83.5±4.6 |
| | 6.4 | 98.2±3.4 | 81.7±3.4 |
| | 3.2 | 96.8±3.1 | 80.3±6.8 |
| Composition G | 12.8 | 119.9±2.4 | 93.9±5.7 |
| | 6.4 | 113.6±5.6 | 90.5±5.3 |
| | 3.2 | 106.4±2.3 | 87.8±3.9 |
| Composition H | 12.8 | 95.4±4.5 | 75.7±3.9 |
| | 6.4 | 94.0±4.8 | 74.6±4.2 |
| | 3.2 | 93.1±4.5 | 73.6±3.0 |
| Composition I | 12.8 | 101.6±3.7 | 83.2±5.8 |
| | 6.4 | 98.2±4.0 | 81.3±4.1 |
| | 3.2 | 96.5±4.7 | 80.4±3.1 |

The data in this table showed that the exhaustive swimming duration of the mice in the ginsenoside composition groups was significantly longer than that in the control group, and the SOD activity was significantly higher than that in the exhaustive swimming group with a significant difference, indicating that the ginsenoside composition has a prominent anti-fatigue effect.

## Claims

1. A method for preparing the composition containing ginsenoside Rg3 and ginsenoside Rg5, which comprises the following steps:

    1) Mix panax ginseng with Chinese medicinal materials;
    2) Decoct the mixture obtained from step 1) with water several times, and collect and combine the decoction fluid;
    3) Add a clarifying agent to the decoction fluid obtained from step 2), mix well, allow to stand and filter;

4) Dry the filtrate obtained from step 3) to obtain the dried substance;

5) Add ethanol aqueous solution to the dried substance obtained from step 4), heat to dissolve, filter, and subject to the low-temperature crystallization process;

6) Dry the crystals obtained from step 5),

Wherein, the Chinese medicinal materials are selected from the groups consisting one or more of the following medicines: mulberry, fried aurantii fructus, paeoniae radix alba, citri sarcodactylis fructus, nelumbinis rhizomatis nodus, fried aurantii fructus immaturus, gingko nut, diospyros kaki calyx, aconitum kusnezofiii leaf, lotus seedpod, ailanthi cortex, Chinese arborvitae twig, punica granatum peel, comus officinalis, gallnut, rhizoma bletillae, white aconite, fructus toosendan, trichosanthis radix, codonopsis pilosula, purple iris, artemisia annua, chinese honeylocust spine, fructus foeniculi, kochia scoparia fruit, peach kernel, mume fructus, herba epimedii, chicken's gizzard-membrane, root of eichmannia, dioscorea oppositifolia, gentiana macrophylla root, adenophora stricta root, pyrrosia lingua, salvia miltiorrhiza, pharbitidis semen, rigonellae semen, lablab purpureus seed, fructus perillae, rhizoma pinelliae preparata, coptis chinensis rhizome, cullen corylifolium fruit, phellodendri chinensis cortex, rhizoma corydafis, canarii fructus, eclipta prostrata, papaya, portulacae herba, citrus medica, achyranthes bidentata, rubus chingii, fallopia multiflora, fraxini cortex, trachycarpus fortunei, nelumbinis semen, agrimonia pilosa herb, green plum, alumen, papaveris pericarpium, terminalia chebula, rosa laevigata, sanguisorba officinalis, prunus dulcis, citrus reticulata pericarp, gardenia jasminoides, ligustri lucidi fructus, rehmannia glutinosa processed root tuber, scutellariae radix, reed rhizome, astragali radix, curcumae rhizoma, mori cortex, ophiopogonis radix, arecae semen, raw frankincense, spatholobi caulis, atractylodis macrocephalae rhizoma, lycii cortex, angelica sinensis, prunus mume smoke treated unripe fruit, hordeum vulgare sprout, eriobotrya japonica leaf, platycladus orientalis seed, cinnamon, asparagi radix, raw myrrh, platycodonis radix, perotis indica, processed radix aconiti lateralis, chrysanthemi flos, aucklandiae radix, radix stemonae, sparganii rhizoma, eucommiae cortex, citrus reticulata fruit peel, rheum officinale, pericarpium arecae, gastrodia elata, pseudostellariae radix, paeonia rubra root, flos inulae, prunella vulgaris, cuscuta chinensis seed, drynaria fortunei, curcumae radix, arisaema cum bile, radix stemonae, taraxacum mongolicum herb, licorice, cimicifugae rhizoma, corydalis bungeana, rubia cordifolia, linderae radix, acanthopanax gracilistylus root bark, aconiti radix, lichee exocarp, emblic leaf flower fruit, belamcandae rhizoma, folium isatidis, euodiae fructus, carthami flos, cyperi rhizoma, schisandra chinensis, ziziphus jujuba seed, crataegi fructus and radix lithospermi.

2. As the method described in Claim 1, wherein, the Chinese medicinal materials are selected from the groups consisting one or more of the following medicines: curcumae radix, arisaema cum bile, radix stemonae, chrysanthemi flos, aucklandiae radix, schisandra chinensis, sparganii rhizoma, atractylodis macrocephalae rhizoma, fructus phyllanthi fruit, pharbitidis semen, phellodendri chinensis cortex, cullen corylifolium fruit, lablab purpureus seed, prunus mume smoke treated unripe fruit, fructus perillae, salvia miltiorrhiza, pyrrosia lingua, dioscorea oppositifolia, root of eichmannia, gentiana macrophylla root, adenophora stricta root, coptis chinensis rhizome, rigonellae semen, rhizoma corydalis, green plum, eucommiae cortex, citrus reticulata fruit peel, ziziphus jujuba seed, gingko nut, punica granatum peel, peach kernel, rhizoma bletillae, cuscuta chinensis seed, crataegi fructus, paeoniae radix alba, Chinese arborvitae twig, pseudostellariae radix, paeonia rubra root, fructus foeniculi, artemisia annua, gallnut, trichosanthis radix, codonopsis pilosula, fructus toosendan, comus officinalis, aurantii fructus immaturus, lotus seedpod, mulberry, chicken's gizzard-membrane, aurantii fructus, ailanthi cortex, aconitum kusnezoffii, michaelmas daisy, chinese honeylocust spine, kochia scoparia fruit, pinelliae rhizoma, rhei radix et rhizoma, Gastrodia elata, flos inulae and prunella vulgaris.

3. As the said method in Claim 1, wherein the weight ratio of panax ginseng to the Chinese medicinal materials is 1:100 to 100: 1, for example, 1: 90-100, 1:45-55, 10: 8-12, 45-55:1 or 90-100:1.

4. As the said method in Claim 1, wherein the number of decoctions is 2-5 times, preferably 3 times.

5. As the said method in Claim 1, wherein the weight of water used for each decoction is 2-100 times, preferably 2-80 times, the total weight of panax ginseng and the Chinese medicinal materials.

6. As the said method in Claim 1, wherein each decoction time is 1-36 h, preferably 2-24 h.

7. As the said method in Claim 1, wherein the temperature of each decoction is 90-100°C, preferably 95-100°C.

8. As the said method in Claim 1, wherein the clarifying agent is the ZTC1+1 clarifying agent.

9. As the said method in Claim 8, wherein the final concentration after the addition of the ZTC1+1 clarifying agent is 200-800 ppm.

10. As the said method in Claim 1, wherein the period of standing is 4-48 h, preferably 4-24 h.

11. As the said method in Claim 1, wherein the aqueous ethanol solution used for crystallization is 40-90% (V/V) aqueous ethanol solution.

12. As the said method in Claim 1, composition is prepared by a process as claimed in any one of claims 1-11.

13. As the said method in Claim 12, wherein the percentage of the total weight of ginsenoside Rg3 and ginsenoside Rg5 to the composition is 1% to 100%, such as 98.0% to 100%.

14. A complex composition includes the composition set forth in Claim 12 or 13 and an acceptable carrier in the industries of pharmaceutics or foods.

15. As the said complex composition in Claim 14, wherein the weight ratio of the composition set forth in Claim 11 to the acceptable carrier in the industries of pharmaceutics or foods is 1:1 to 1:100.

16. As the said complex composition in Claim 14, it may exist in the forms of tablets, capsules, pills, powder, granules, syrup, solution, emulsion, spray, aerosol, gel, cream, cataplasm, adhesive plaster or emplastrums, or in the forms of foods such as dairy products, confectionery, beverages, biscuits, tea leaves and related products, wine and the like.

17. The application of the said composition in Claim 12 or 13 in the preparation of drug products for boosting immunity, enhancing anti-tumor effect, improving resistance to anti-tumor targeted drugs, mitigating toxic and side effects of radiotherapy and chemotherapy or improving anti-fatigue effect.

18. A method for boosting immunity, enhancing anti-tumor effect, improving resistance to anti-tumor targeted drugs, mitigating toxic and side effects of radiotherapy and chemotherapy or improving anti-fatigue effect, which comprises the administration of an effective amount of the composition set forth in Claim 12 or 13 to an individual who need the composition.

## INTERNATIONAL SEARCH REPORT

| | International application No. |
|---|---|
| | **PCT/CN2021/113658** |

### A. CLASSIFICATION OF SUBJECT MATTER

A61K 31/704(2006.01)i;  A61K 36/904(2006.01)i;  A61K 36/899(2006.01)i;  A61K 36/8988(2006.01)i;  A61K 36/898(2006.01)i;  A61K 36/8968(2006.01)i;  A61K 36/8966(2006.01)i;  A61K 36/8965(2006.01)i;  A61K 36/8945(2006.01)i;  A61K 36/725(2006.01)i;  A61K 36/57(2006.01)i;  A61K 36/258(2006.01)i;  A61P 37/04(2006.01)i;  A61P 35/00(2006.01)i;  A61P 39/00(2006.01)i;  A61K 35/64(2015.01)i;  A61K 36/734(2006.01)i

According to International Patent Classification (IPC) or to both national classification and IPC

### B. FIELDS SEARCHED

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNABS, DWPI, SIPOABS, CNTXT, 中国药物专利数据库(网页), VEN, CNKI, 药品标准数据库(E药全库和国家新药注册数据), PUBMED: 百部, 芦根, 天麻, 白及, 麦冬, 麦门冬, 贝母, 天冬, 天门冬, 山药, 淮山, 大枣, 枣, 五味子, 山楂, 人参, 人参皂苷, 水, 澄清剂, 乙醇, 酒精, 冷却, 析出, 结晶, 免疫, 肿瘤, 放疗, 化疗, 抗疲劳, Radix Stemonae, Rhizoma Phragmitis, Rhizoma Gastrodiae, Bletillae, Radix Ophiopogonis, Bulbus Fritillariae Thunbergii, Radix Asparagi, Rhizoma Dioscoreae, Fructus Jujubae, Schisandrae, Fructus Crataegi, Ginseng, ginsenoside, Water, Clarifying Agents, Ethanol, Alcohol, Cooling, Precipitation, Crystallization, Immunology, Tumor, Radiotherapy, Chemotherapy, Anti-Fatigue

### C. DOCUMENTS CONSIDERED TO BE RELEVANT

| Category* | Citation of document, with indication, where appropriate, of the relevant passages | Relevant to claim No. |
|---|---|---|
| PX | CN 112245443 A (FU, Li) 22 January 2021 (2021-01-22)<br>description, paragraphs [0019]-[0045] | 1-17 |
| X | 郑飞 等 (ZHENG, Fei et al.). "高效液相色谱-飞行时间质谱联用法分析人参及人参皂苷与山楂配伍过程中的水解行为 (Hydrolysis Behaviors of Combined Dectotion of Panax ginseng and Ginsenosides with Hawthorn by HPLC-Q-TOF MS/MS)"<br>应用化学 (Chinese Journal of Applied Chemistry),<br>Vol. 34, No. 6, 10 June 2017 (2017-06-10),<br>pp. 723-728 | 1-17 |
| A | CN 109316517 A (LIAONING XIFENG PHARMACY CO., LTD.) 12 February 2019 (2019-02-12)<br>claim 1, and abstract | 1-17 |

☑ Further documents are listed in the continuation of Box C.          ☑ See patent family annex.

| | |
|---|---|
| \*    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **22 September 2021** | **04 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/113658** |

**C.** **DOCUMENTS CONSIDERED TO BE RELEVANT**

| Category* | Citation of document, with indication, where appropriate, of the relevant passages | Relevant to claim No. |
| --- | --- | --- |
| A | 章春芳 (ZHANG, Chunfang). "低极性人参皂甙的制备研究 (Preparation on the Ginsenoside with Low Polar)" 安徽化工 (Anhui Chemical Industry), No. 3, 30 June 2005 (2005-06-30), p. 25 | 1-17 |

Form PCT/ISA/210 (second sheet) (January 2015)

**INTERNATIONAL SEARCH REPORT**

| International application No. |
| --- |
| **PCT/CN2021/113658** |

| Box No. II    Observations where certain claims were found unsearchable (Continuation of item 2 of first sheet) |
| --- |

This international search report has not been established in respect of certain claims under Article 17(2)(a) for the following reasons:

1. ☑ Claims Nos.: **18**
because they relate to subject matter not required to be searched by this Authority, namely:

   [1]  The subject matter of claim 18 relates to treatment methods for the human or animal body, and does not comply with PCT Rule 39.1 (iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2021/113658**

| Patent document cited in search report | | | Publication date (day/month/year) | Patent family member(s) | | | Publication date (day/month/year) |
|---|---|---|---|---|---|---|---|
| CN | 112245443 | A | 22 January 2021 | None | | | |
| CN | 109316517 | A | 12 February 2019 | CN | 109316517 | B | 13 April 2021 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Handbook of Pharmaceutical Excipients. American Pharmaceutical Association, Washington and The Pharmaceutical Press, 1994